# EUROPEAN PATENT APPLICATION

(11) **EP 4 596 579 A1**
(43) Date of publication of application: **06.08.2025**
(21) Application number: 22959915.4
(22) Date of filing: 28.09.2022
(51) Int. Cl.: C07K 16/22, C07K 16/46, C07K 19/00, C12N 15/13, C12N 15/62, C12N 5/10, C12N 15/63, A61K 39/395, A61P 27/02, A61P 29/00, A61P 35/00

(54) **ANTIBODY OR ANTIGEN-BINDING FRAGMENT CAPABLE OF IDENTIFYING ANG-2, AND BISPECIFIC ANTIBODY FOR SIMULTANEOUSLY IDENTIFYING VEGF AND ANG-2**

(71) Applicant: Kexing Biopharm Co., Ltd., Jinan, Shandong 250200 (CN)
(72) Inventor: WEI, Xiling, Jinan, Shandong 250200 (CN); QIN, Suofu, Jinan, Shandong 250200 (CN); QIU, Yuxin, Jinan, Shandong 250200 (CN); SHANG, Wei, Jinan, Shandong 250200 (CN)
(74) Representative: Nederlandsch Octrooibureau
(86) International application number: PCT/CN2022/122112
(87) International publication number: WO 2024/065268

(57) **Abstract**

Provided is a single-domain antibody or antigen-binding fragment capable of recognizing ANG-2, and a multispecific antibody. The antibody or antigen-binding fragment has a heavy chain variable region as represented by any one of SEQ ID NO: 16 to SEQ ID NO: 20. The multispecific antibody comprises a first binding region containing the aforementioned antibody or antigen-binding fragment, and a second binding region with binding activity to a first molecule.

## Description

### FIELD

The present disclosure belongs to the field of biopharmaceutical technologies, and particularly relates to an antibody or antigen-binding fragment capable of recognizing ANG-2, as well as a bispecific antibody or an antibody fusion protein simultaneously recognizing VEGF and ANG-2, more particularly, to an antibody or antigen-binding fragment, recombinant protein, multispecific antibody, nucleic acid molecule, expression vector, recombinant cell, conjugate, pharmaceutical composition, kit and uses thereof, as well as a method for treating or preventing an angiogenesis-related disease, a method for diagnosing and evaluating an angiogenesis-related disease, and a method for assessing the prognosis of an angiogenesis-related disease.

### BACKGROUND

Current studies indicate that angiogenesis plays a crucial role in the pathogenesis various diseases, including cancers, intraocular neovascular syndromes (e.g., proliferative retinopathies), age-related macular degeneration (AMD), and inflammatory diseases (e.g., rheumatoid arthritis and psoriasis).

Angiopoietin-2 (ANG-2) is a secreted glycoprotein with a molecular weight of about 70 kDa, which plays complex roles in angiogenesis, inflammation, and vascular development. It is expressed in vascular endothelial cells, smooth muscle cells of highly angiogenic tissues, pulmonary epithelial cells, differentiated myotubes, and neural progenitor cells. ANG-2, upon binding to its receptor TEK tyrosine kinase (TIE2), promotes the survival, proliferation and migration of endothelial cells, drives sprouting angiogenesis, induces the loss of perivascular cells, and enhances vascular permeability. Therefore, the inhibition of the ANG-2 signaling pathway can treat angiogenesis-related diseases including tumors and ophthalmic diseases.

Therefore, developing antibodies with enhanced binding activities to ANG-2 is important for the treatment of angiogenesis-related diseases.

### SUMMARY

This disclosure aims to address, at least to some extent, one of the technical problems existing in the prior art. To achieve this, this disclosure provides an anti-ANG-2 single-domain antibody, which has high binding activity to ANG-2, exerts anti-angiogenic effects and reduces vascular inflammation by blocking the interaction between ANG-2 and TIE2.

This disclosure is based on the following findings of the inventors.

Drugs developed to inhibit ANG-2 activity include anti-ANG-2 monoclonal antibodies (WO2013/144266) and soluble ANG-2 receptor inhibitors, such as soluble TIE2 (Siemeister, G., et al., Cancer Res, 59:3 (1999), 3185-91). However, single-domain antibody drugs that inhibit the ANG-2 activity are relatively scarce.

Compared with conventional antibodies, single-domain antibodies (also known as nanobodies, Nbs) offer several advantages, including: 1) small molecular weight, enabling penetration of the blood-brain barrier; 2) high expression levels in both prokaryotic or eukaryotic systems; 3) high specificity and strong affinity; 4) low immunogenicity in humans. Consequently, single-domain antibodies are widely applied in biopharmaceutical research and development.

In view of the above, the inventors immunized alpacas with recombinant ANG-2 protein, constructed and screened a phage display library using peripheral blood collected from the immunized alpacas, and finally obtained a single-domain antibody with high affinity for ANG-2. Furthermore, the single-domain antibody and VEGF antibody/VEGF receptor can be used to develop a bispecific antibody (hereinafter referred to as "bsAb") or an antibody fusion protein, which can simultaneously bind to VEGF and ANG-2. The bsAb inhibits the interaction of VEGF with VEGFR andANG-2 with TIE2, thereby exerting anti-angiogenic effects and reducing vascular inflammation.

Therefore, one aspect of this disclosure provides an antibody or antigen-binding fragment. According to an embodiment of the present disclosure, the antibody or antigen-binding fragment includes at least one heavy chain variable region CDR sequence selected from SED ID NO: 1 to SED ID NO: 14 or an amino acid sequence having at least 80% identity to SED ID NO: 1 to SED ID NO: 14. The above-mentioned antibody or antigen-binding fragment of the present disclosure can target and bind to ANG-2 with high binding activity, and exerts anti-angiogenesis effects and reduces vascular inflammations by blocking the binding of ANG-2 to TIE2. Therefore, it can be effectively used in the treatment or prevention of angiogenesis-related diseases.

According to an embodiment of the present disclosure, the antibody or antigen-binding fragment may further include at least one of the following additional technical features.

According to an embodiment of the present disclosure, the antibody or antigen-binding fragment includes:
a heavy chain variable region CDR1 sequence, a heavy chain variable region CDR2 sequence, and a heavy chain variable region CDR3 sequence specified in amino acid sequences SEQ ID NO: 1, SED ID NO: 2, and SED ID NO: 3, respectively, or with amino acid sequences having at least 80% identity to SEQ ID NO: 1, SED ID NO: 2, and SED ID NO: 3, respectively;
a heavy chain variable region CDR1 sequence, a heavy chain variable region CDR2 sequence, and a heavy chain variable region CDR3 sequence specified in amino acid sequences SEQ ID NO: 4, SED ID NO: 5, and SED ID NO: 6, respectively, or with amino acid sequences having at least 80% identity to SEQ ID NO: 4, SEQ ID NO: 5, and SEQ ID NO: 6, respectively;
a heavy chain variable region CDR1 sequence, a heavy chain variable region CDR2 sequence, and a heavy chain variable region CDR3 sequence specified in amino acid sequences SEQ ID NO: 7, SED ID NO: 8, and SED ID NO: 9, respectively, or with amino acid sequences having at least 80% identity to SEQ ID NO: 7, SEQ ID NO: 8, and SEQ ID NO: 9, respectively;
a heavy chain variable region CDR1 sequence, a heavy chain variable region CDR2 sequence, and a heavy chain variable region CDR3 sequence specified in amino acid sequences SEQ ID NO: 10, SED ID NO: 11, and SED ID NO: 12, respectively, or with amino acid sequences having at least 80% identity to SEQ ID NO: 10, SED ID NO: 11, and SED ID NO: 12, respectively; or
a heavy chain variable region CDR1 sequence, a heavy chain variable region CDR2 sequence, and a heavy chain variable region CDR3 sequence specified in amino acid sequences SEQ ID NO: 10, SED ID NO: 13, and SED ID NO: 14, respectively, or with amino acid sequences having at least 80% identity to SEQ ID NO: 10, SED ID NO: 13, and SED ID NO: 14, respectively.
YYSIG (SEQ ID NO: 1);
CISRTDGSTYYADSVKG (SEQ ID NO: 2);
KGARYSGSYLCGLDEYDY (SEQ ID NO: 3);
SATMS (SEQ ID NO: 4);
LIAYAGGSTTYADSVKG (SEQ ID NO: 5);
RRYNRDF (SEQ ID NO: 6);
YYAIG (SEQ ID NO: 7);
CISSGDGSTYYADSVKG (SEQ ID NO: 8);
VRSGSKCLVTRGTVVAAGQLYEY (SEQ ID NO: 9);
FSTVR (SEQ ID NO: 10);
TLLDDGSTNYADSVKG (SEQ ID NO: 11);
DGRTGWGPRGDY (SEQ ID NO: 12);
THDDGSTNYEDSVKG (SEQ ID NO: 13);
DGSTGWGPRGDY (SEQ ID NO: 14).

According to an embodiment of the present disclosure, the antibody includes a heavy chain framework region sequence, wherein at least a part of the heavy chain framework region sequence is derived from at least one of murine antibody, human antibody, primate antibody, alpaca antibody, or a variant thereof.

According to an embodiment of the present disclosure, the antibody has a heavy chain variable region with an amino acid sequence specified in any one of SEQ ID NO: 16 to SEQ ID NO: 20.

QLQLVESGGGLVQPGGSLRLSCAASGFTLGYYSIGWFRQAPGKEREGVSCI
SRTDGSTYYADSVKGRFTISRDNAKNTVYLQMNNLKPEDTAVYYCAVKGARYSGSYL
CGLDEYDYWGQGTQVTVSS (SEQ ID NO: 16);

QVQLVESGGGLVQPGGSLRLSCAASGFSFSSATMSWHRQTPGKERTMVALI

AYAGGSTTYADSVKGRFTISRDNAKNTVYLQMSNLKPEDTAVYYCNTRRYNRDFWG
QGTQVTVSS (SEQ ID NO: 17);

QLQLVESGGGLVQPGGSLRLSCAASGFTLDYYAIGWFRQVPGKAREGLSCI
SSGDGSTYYADSVKGRFTISRDNTKNTVYLQMNSLKPEDTAVYSCAAVRSGSKCLVT
RGTVVAAGQLYEYWGQGTQVTVSS (SEQ ID NO: 18);

QVQLVESGGGLVQPGGSLRLSCAASGSIFSFSTVRWYRQAPGKQREPVATL
LDDGSTNYADSVKGRFTISTDNAKNTVYLQMNSLKPEDTAVYYCNADGRTGWGPRG
DYWGRGTQVTVSS (SEQ ID NO: 19);

QVQLVESGGGLVQPGGSLRLSCAASGSSFKFSTVRWYRQAPGKQREPVAT
HDDGSTNYEDSVKGRFTISRDNAKNTAFLQMNSLKPEDTAVYYCNADGSTGWGPRG
DYWGRGTQVTVSS (SEQ ID NO: 20).

According to an embodiment of the present disclosure, the antibody includes a heavy chain constant region, and at least a part of the heavy chain constant region is derived from at least one of murine antibody, human antibody, primate antibody, or a variant thereof.

According to an embodiment of the present disclosure, the heavy chain constant region is an Fc fragment.

According to an embodiment of the present disclosure, the heavy chain constant region of the antibody is derived from a human IgG antibody or a variant thereof.

According to an embodiment of the present disclosure, the heavy chain constant region of the antibody is derived from human IgG1.

According to an embodiment of the present disclosure, the antibody is a monoclonal antibody or a polyclonal antibody.

According to an embodiment of the present disclosure, the monoclonal antibody includes at least one of Fab antibody, Fv antibody, single-chain antibody, single-domain antibody, and minimal recognition unit.

According to a preferred example of the present disclosure, the antibody is a single-domain antibody.

According to an embodiment of the present disclosure, the antibody has a heavy chain with an amino acid sequence specified in any one of SEQ ID NO: 21 to SEQ ID NO: 25.

QLQLVESGGGLVQPGGSLRLSCAASGFTLGYYSIGWFRQAPGKEREGVSCI

SRTDGSTYYADSVKGRFTISRDNAKNTVYLQMNNLKPEDTAVYYCAVKGARYSGSYL
CGLDEYDYWGQGTQVTVSSEPKSADKTHTCPPCPAPELLGGPSVFLFPPKPKDTLMIS
RTPEVTCVVVDVSHEDPEVKFNWYVDGVEVHNAKTKPREEQYNSTYRVVSVLTVLH
QDWLNGKEYKCKVSNKALPAPIEKTISKAKGQPREPQVYTLPPSREEMTKNQVSLTC
LVKGFYPSDIAVEWESNGQPENNYKTTPPVLDSDGSFFLYSKLTVDKSRWQQGNVFSC
SVMHEALHNHYTQKSLSLSPGK (SEQ ID NO: 21);

QVQLVESGGGLVQPGGSLRLSCAASGFSFSSATMSWHRQTPGKERTMVALI
AYAGGSTTYADSVKGRFTISRDNAKNTVYLQMSNLKPEDTAVYYCNTRRYNRDFWG
QGTQVTVSSEPKSADKTHTCPPCPAPELLGGPSVFLFPPKPKDTLMISRTPEVTCVVVD
VSHEDPEVKFNWYVDGVEVHNAKTKPREEQYNSTYRVVSVLTVLHQDWLNGKEYK
CKVSNKALPAPIEKTISKAKGQPREPQVYTLPPSREEMTKNQVSLTCLVKGFYPSDIAV
EWESNGQPENNYKTTPPVLDSDGSFFLYSKLTVDKSRWQQGNVFSCSVMHEALHNH
YTQKSLSLSPGK (SEQ ID NO: 22);

QLQLVESGGGLVQPGGSLRLSCAASGFTLDYYAIGWFRQVPGKAREGLSCI
SSGDGSTYYADSVKGRFTISRDNTKNTVYLQMNSLKPEDTAVYSCAAVRSGSKCLVT
RGTVVAAGQLYEYWGQGTQVTVSSEPKSADKTHTCPPCPAPELLGGPSVFLFPPKPK
DTLMISRTPEVTCVVVDVSHEDPEVKFNWYVDGVEVHNAKTKPREEQYNSTYRVVS
VLTVLHQDWLNGKEYKCKVSNKALPAPIEKTISKAKGQPREPQVYTLPPSREEMTKN
QVSLTCLVKGFYPSDIAVEWESNGQPENNYKTTPPVLDSDGSFFLYSKLTVDKSRWQQ
GNVFSCSVMHEALHNHYTQKSLSLSPGK (SEQ ID NO: 23);

QVQLVESGGGLVQPGGSLRLSCAASGSIFSFSTVRWYRQAPGKQREPVATL
LDDGSTNYADSVKGRFTISTDNAKNTVYLQMNSLKPEDTAVYYCNADGRTGWGPRG
DYWGRGTQVTVSSEPKSADKTHTCPPCPAPELLGGPSVFLFPPKPKDTLMISRTPEVT
CVVVDVSHEDPEVKFNWYVDGVEVHNAKTKPREEQYNSTYRVVSVLTVLHQDWLN
GKEYKCKVSNKALPAPIEKTISKAKGQPREPQVYTLPPSREEMTKNQVSLTCLVKGFY
PSDIAVEWESNGQPENNYKTTPPVLDSDGSFFLYSKLTVDKSRWQQGNVFSCSVMHE
ALHNHYTQKSLSLSPGK (SEQ ID NO: 24);

QVQLVESGGGLVQPGGSLRLSCAASGSSFKFSTVRWYRQAPGKQREPVAT
HDDGSTNYEDSVKGRFTISRDNAKNTAFLQMNSLKPEDTAVYYCNADGSTGWGPRG
DYWGRGTQVTVSSEPKSADKTHTCPPCPAPELLGGPSVFLFPPKPKDTLMISRTPEVT

CVVVDVSHEDPEVKFNWYVDGVEVHNAKTKPREEQYNSTYRVVSVLTVLHQDWLN
GKEYKCKVSNKALPAPIEKTISKAKGQPREPQVYTLPPSREEMTKNQVSLTCLVKGFY
PSDIAVEWESNGQPENNYKTTPPVLDSDGSFFLYSKLTVDKSRWQQGNVFSCSVMHE
ALHNHYTQKSLSLSPGK (SEQ ID NO: 25).

In another aspect of the present disclosure, the present disclosure provides a recombinant protein. According to an embodiment of the present disclosure, the recombinant protein includes the above-mentioned antibody or antigen-binding fragment. The recombinant protein according to an embodiment of the present disclosure can target and bind to ANG-2 with high ANG-2 binding activity, can be used to detect ANG-2, and exerts anti-angiogenesis effects and reduces vascular inflammations by blocking the binding of ANG-2 to TIE2. Therefore, it can be effectively used in the treatment or prevention of angiogenesis-related diseases.

According to an embodiment of the present disclosure, the recombinant protein further includes at least one of a bioactive protein or fragment thereof, and a bioactive polypeptide or fragment thereof.

According to an embodiment of the present disclosure, the bioactive protein or fragment thereof includes at least one selected from a protein tag, a protein toxin or fragment thereof, a tumor necrosis factor or fragment thereof, an interferon or fragment thereof, a biological response regulator or fragment thereof, and an Fc fragment.

The term "protein tag" used herein generally refers to a polypeptide or protein that is fused and co-expressed with a target protein (the antibody or antigen-binding fragment). It is useful for the expression, detection, tracking or purification of the target protein. Examples of protein tags include, but are not limited to, His tag (also known as His-Tag, with the sequence HHHHHH), Flag tag (also known as Flag-Tag, with the sequence DYKDDDDK), GST tag (also known as GST-Tag, glutathione S-transferase tag), MBP tag (also known as MBP-Tag, maltose-binding protein tag), SUMO tag, and C-Myc tag.

The term "toxin" used herein generally refers to substances that are toxic to a host, including protein toxins and non-protein toxins. Protein toxins include, but are not limited to, abrin, ricin A chain, Pseudomonas exotoxin, and diphtheria toxin. In the present disclosure, the protein toxin is preferably a protein toxin having enzymatic activity.

The term "tumor necrosis factor" used herein generally refers to substances that can cause hemorrhagic necrosis in a variety of tumors, including but not limited to TNF-α and TNF-β.

The term "interferon" used herein generally refers to a glycoprotein that has the ability to directly kill or inhibit viruses. The interferon includes, but is not limited to, IFN-α, INF-β, and IFN-γ.

The term "biological response regulator" used herein generally refers to a class of protein substances that directly or indirectly enhance the body's anti-tumor effects through the immune system. Examples include, but are not limited to, lymphokines, IL-2, IL-6, IL-10, and GM-CSF.

The term "Fc fragment" used herein generally refers to an Fc region from IgG (e.g., IgG1, IgG2, IgG3, or IgG4 subtype), IgA1, IgA2, IgD, IgE, or IgM, including the CH2 and CH3 domains, and optionally the hinge region. Preferably, IgG, IgA1, IgA2, IgD, IgE, or IgM is derived from murine, human, primate, or alpaca.

In another aspect of the present disclosure, the present disclosure provides a multispecific antibody. According to an embodiment of the present disclosure, the multispecific antibody includes: a first binding region including the above-mentioned antibody or antigen-binding fragment; and a second binding region having an activity of binding to a first molecule. The multispecific antibody of the present disclosure is capable of simultaneously binding to both ANG-2 and the first molecule, can be used to detect ANG-2 and the first molecule, and exerts anti-angiogenesis effects and reduces vascular inflammations by blocking the binding of ANG-2 to TIE2. Therefore, it can be effectively used in the treatment or prevention of angiogenesis-related diseases.

According to an embodiment of the present disclosure, the multispecific antibody may further include at least one of the following additional technical features.

According to an embodiment of the present disclosure, the multispecific antibody includes at least one selected from a bispecific antibody, a trispecific antibody, and a tetraspecific antibody, preferably a bispecific antibody.

It should be noted that the number of the second binding region may be one or more than one. When the multispecific antibody is a bispecific antibody, the number of the second binding region is one; when the multispecific antibody is a trispecific antibody, the number of the second binding region is two, each capable of binding to a different first molecule; and when the multispecific antibody is a tetraspecific antibody, the number of the second binding region is three, each capable of binding to a different first molecule.

According to an embodiment of the present disclosure, the first molecule includes at least one selected from immune cell surface antigen, tumor antigen, virus, bacteria, endotoxin, cytokine, and cytokine receptor.

The term "immune cell surface antigen" used herein should be interpreted broadly to refer to proteins with immunogenicity on the surfaces of immune cells (e.g., T cells, NK cells, or B cells). Examples include, but are not limited to, BCMA, CTLA-4, LAG-3, TIGIT, CD38, SLAMF7, B7-H3, CD19, CD20, CD30, CD33, CD47, CD52, CD133, RANKL, CD3, and CD16a.

The term "tumor antigen" used herein should be interpreted broadly to refer to proteins with immunogenicity on the surfaces of tumor cells. Examples include, but are not limited to, PD-L1, PD-1, TGF-β, CEA, GD2, and GD3.

The term "cytokine" used herein should be interpreted broadly to refer to a class of proteins or small molecule peptides that mediate intercellular signaling and possess immunoregulatory and effector functions. Examples include, but are not limited to, IL-10, VEGF (including at least one of VEGF-A, VEGF-B, VEGF-C, VEGF-D, VEGF-E, VEGF-F, and PIGF), EpCAM, GM2, and RANKL.

The term "cytokine receptors" used herein should be interpreted broadly to refer to receptors on the surfaces of cells that can bind to cytokines. Examples include, but are not limited to, Her2, EGFR, and IL-10R.

According to an embodiment of the present disclosure, the first molecule comprises at least one selected from PD-L1, PD-1, IL-10, IL-10R, BCMA, VEGF, TGF-β, CTLA-4, LAG-3, TIGIT, CEA, CD38, SLAMF7, B7-H3, Her2, EpCAM, CD19, CD20, CD30, CD33, CD47, CD52, CD133, EGFR, GD2, GD3, GM2, RANKL, CD3, and CD16a.

According to an embodiment of the present disclosure, the second binding region is a binding protein or fragment thereof to the first molecule.

It should be noted that the term "binding protein or fragment thereof to the first molecule" generally refers to a protein or protein fragment thereof capable of binding to the first molecule. When the first molecule is a protein receptor, such as IL-10R and EGFR, the binding protein or fragment thereof to the first molecule may be a cytokine (IL-10 or EGF) or fragment thereof, or an antibody or fragment thereof that binds to the protein receptor; when the first molecule is a protein other than a protein receptor, such as IL-10 and VEGF, the binding protein or fragment thereof to the first molecule may be a protein receptor (IL-10R and VEGFR) or fragment thereof, or an antibody or fragment thereof that binds to this protein.

According to an embodiment of the present disclosure, the first molecule is VEGF. The multispecific antibody of the present disclosure is capable of simultaneously binding to both VEGF and ANG-2, and exerts anti-angiogenesis effects and reduces vascular inflammations by blocking the binding of VEGF to VEGFR and the binding of ANG-2 to TIE2. Therefore, it can be effectively used in the treatment or prevention of angiogenesis-related diseases.

According to an embodiment of the present disclosure, the second binding region includes at least one selected from a VEGF receptor fragment and an anti-VEGF antibody.

According to an embodiment of the present disclosure, the VEGF receptor fragment includes at least one selected from a VEGFR1 fragment, a VEGFR2 fragment, and a VEGFR3 fragment.

It should be noted that the VEGF receptor (abbreviated as VEGFR) consists of three parts: an extracellular VEGF-binding region, a transmembrane (TM) region of the receptor, and an intracellular signaling domain. Specifically, the VEGF-binding region is composed of seven immunoglobulin-like domains, which are individually referred to as immunoglobulin-like domain 1 to immunoglobulin-like domain 7 (also known as D1 to D7).

According to an embodiment of the present disclosure, the second binding region includes an immunoglobulin-like domain 2 of the VEGFR1 fragment and/or an immunoglobulin-like domain 3 of the VEGFR2 fragment.

According to an embodiment of the present disclosure, the C-terminus of immunoglobulin-like domain 2 of the VEGFR1 fragment is linked to the N-terminus of immunoglobulin-like domain 3 of the VEGFR2 fragment.

According to an embodiment of the present disclosure, the bispecific antibody is a symmetric bispecific antibody or an asymmetric bispecific antibody.

According to an embodiment of the present disclosure, the bispecific antibody is a symmetric bispecific antibody.

According to an embodiment of the present disclosure, the second binding region further includes an Fc fragment.

According to an embodiment of the present disclosure, the Fc fragment includes CH2 region, CH3 region and optionally a hinge region.

According to an embodiment of the present disclosure, the C-terminus of the CH2 region is linked to the N-terminus of the CH3 region.

According to an embodiment of the present disclosure, the C-terminus of the hinge region is linked to the N-terminus of the CH2 region, and the C-terminus of the CH2 region is linked to the N-terminus of the CH3 region.

According to an embodiment of the present disclosure, at least a part of the Fc fragment is derived from at least one of murine antibody, human antibody, primate antibody, or a variant thereof.

According to an embodiment of the present disclosure, at least a part of the Fc fragment is derived from a human antibody or a variant thereof.

According to an embodiment of the present disclosure, at least a part of the Fc fragment is derived from human antibody IgG or a variant thereof.

According to an embodiment of the present disclosure, the C-terminus of immunoglobulin-like domain 2 of the VEGFR1 fragment is linked to the N-terminus of immunoglobulin-like domain 3 of the VEGFR2 fragment and the C-terminus of immunoglobulin-like domain 3 of the VEGFR2 fragment is linked to the N-terminus of the Fc fragment. Alternatively, the C-terminus of immunoglobulin-like domain 2 of the VEGFR1 fragment is linked to the N-terminus of immunoglobulin-like domain 3 of the VEGFR2 fragment, and the N-terminus of immunoglobulin-like domain 2 in the VEGFR1 fragment is linked to the C-terminus of the Fc fragment.

According to an embodiment of the present disclosure, the second binding region has an amino acid sequence specified in SEQ ID NO: 26 or an amino acid sequence having at least 90% identity to SEQ ID NO: 26.

SDTGRPFVEMYSEIPEIIHMTEGRELVIPCRVTSPNITVTLKKFPLDTLIPDGK
RIIWDSRKGFIISNATYKEIGLLTCEATVNGHLYKTNYLTHRQTNTIIDVVLSPSHGIELS
VGEKLVLNCTARTELNVGIDFNWEYPSSKHQHKKLVNRDLKTQSGSEMKKFLSTLTI
DGVTRSDQGLYTCAASSGLMTKKNSTFVRVHEKDKTHTCPPCPAPELLGGPSVFLFPP
KPKDTLMISRTPEVTCVVVDVSHEDPEVKFNWYVDGVEVHNAKTKPREEQYNSTYR
VVSVLTVLHQDWLNGKEYKCKVSNKALPAPIEKTISKAKGQPREPQVYTLPPSRDELT
KNQVSLTCLVKGFYPSDIAVEWESNGQPENNYKTTPPVLDSDGSFFLYSKLTVDKSRW
QQGNVFSCSVMHEALHNHYTQKSLSLSPG (SEQ ID NO: 26).

According to an embodiment of the present disclosure, the multispecific antibody further includes a linking peptide.

According to an embodiment of the present disclosure, the C-terminus of immunoglobulin-like domain 3 of the VEGFR2 fragment is linked to the N-terminus of the Fc fragment, the C-terminus of the Fc fragment is linked to the N-terminus of the linking peptide, and the C-terminus of the linking peptide is linked to the N-terminus of the antibody or antigen-binding fragment. Alternatively, the C-terminus of the antibody or antigen-binding fragment is linked to the N-terminus of the linking peptide, the C-terminus of the linking peptide is linked to the N-terminus of the Fc fragment, and the C-terminus of the Fc fragment is linked to the N-terminus of immunoglobulin-like domain 2 of the VEGFR1 fragment.

According to an embodiment of the present disclosure, the amino acid sequence of the linking peptide is (GGGGS)ₙ, where n is an integer greater than or equal to 1, preferably 1, 2, 3, 4, 5, 6, 7, 8, 9 or 10.

According to an embodiment of the present disclosure, the linking peptide has an amino acid sequence specified in SEQ ID NO: 27.

GGGGSGGGGSGGGGSGGGGS (SEQ ID NO: 27).

According to an embodiment of the present disclosure, the antibody has an amino acid sequence specified in any one of SEQ ID NO: 28 to SEQ ID NO: 32.

SDTGRPFVEMYSEIPEIIHMTEGRELVIPCRVTSPNITVTLKKFPLDTLIPDGK
RIIWDSRKGFIISNATYKEIGLLTCEATVNGHLYKTNYLTHRQTNTIIDVVLSPSHGIELS
VGEKLVLNCTARTELNVGIDFNWEYPSSKHQHKKLVNRDLKTQSGSEMKKFLSTLTI
DGVTRSDQGLYTCAASSGLMTKKNSTFVRVHEKDKTHTCPPCPAPELLGGPSVFLFPP

KPKDTLMISRTPEVTCVVVDVSHEDPEVKFNWYVDGVEVHNAKTKPREEQYNSTYR
VVSVLTVLHQDWLNGKEYKCKVSNKALPAPIEKTISKAKGQPREPQVYTLPPSRDELT
KNQVSLTCLVKGFYPSDIAVEWESNGQPENNYKTTPPVLDSDGSFFLYSKLTVDKSRW
QQGNVFSCSVMHEALHNHYTQKSLSLSPGGGGGSGGGGSGGGGSGGGGSQLQLVES
GGGLVQPGGSLRLSCAASGFTLGYYSIGWFRQAPGKEREGVSCISRTDGSTYYADSVK
GRFTISRDNAKNTVYLQMNNLKPEDTAVYYCAVKGARYSGSYLCGLDEYDYWGQG
TQVTVSS (SEQ ID NO: 28);

SDTGRPFVEMYSEIPEIIHMTEGRELVIPCRVTSPNITVTLKKFPLDTLIPDGK
RIIWDSRKGFIISNATYKEIGLLTCEATVNGHLYKTNYLTHRQTNTIIDVVLSPSHGIELS
VGEKLVLNCTARTELNVGIDFNWEYPSSKHQHKKLVNRDLKTQSGSEMKKFLSTLTI
DGVTRSDQGLYTCAASSGLMTKKNSTFVRVHEKDKTHTCPPCPAPELLGGPSVFLFPP
KPKDTLMISRTPEVTCVVVDVSHEDPEVKFNWYVDGVEVHNAKTKPREEQYNSTYR
VVSVLTVLHQDWLNGKEYKCKVSNKALPAPIEKTISKAKGQPREPQVYTLPPSRDELT
KNQVSLTCLVKGFYPSDIAVEWESNGQPENNYKTTPPVLDSDGSFFLYSKLTVDKSRW
QQGNVFSCSVMHEALHNHYTQKSLSLSPGGGGGSGGGGSGGGGSGGGGSQVQLVES
GGGLVQPGGSLRLSCAASGFSFSSATMSWHRQTPGKERTMVALIAYAGGSTTYADSV
KGRFTISRDNAKNTVYLQMSNLKPEDTAVYYCNTRRYNRDFWGQGTQVTVSS (SEQ
ID NO: 29);

SDTGRPFVEMYSEIPEIIHMTEGRELVIPCRVTSPNITVTLKKFPLDTLIPDGK
RIIWDSRKGFIISNATYKEIGLLTCEATVNGHLYKTNYLTHRQTNTIIDVVLSPSHGIELS
VGEKLVLNCTARTELNVGIDFNWEYPSSKHQHKKLVNRDLKTQSGSEMKKFLSTLTI
DGVTRSDQGLYTCAASSGLMTKKNSTFVRVHEKDKTHTCPPCPAPELLGGPSVFLFPP
KPKDTLMISRTPEVTCVVVDVSHEDPEVKFNWYVDGVEVHNAKTKPREEQYNSTYR
VVSVLTVLHQDWLNGKEYKCKVSNKALPAPIEKTISKAKGQPREPQVYTLPPSRDELT
KNQVSLTCLVKGFYPSDIAVEWESNGQPENNYKTTPPVLDSDGSFFLYSKLTVDKSRW
QQGNVFSCSVMHEALHNHYTQKSLSLSPGGGGGSGGGGSGGGGSGGGGSQLQLVES
GGGLVQPGGSLRLSCAASGFTLDYYAIGWFRQVPGKAREGLSCISSGDGSTYYADSV
KGRFTISRDNTKNTVYLQMNSLKPEDTAVYSCAAVRSGSKCLVTRGTVVAAGQLYEY
WGQGTQVTVSS (SEQ ID NO: 30);

SDTGRPFVEMYSEIPEIIHMTEGRELVIPCRVTSPNITVTLKKFPLDTLIPDGK

RIIWDSRKGFIISNATYKEIGLLTCEATVNGHLYKTNYLTHRQTNTIIDVVLSPSHGIELS
VGEKLVLNCTARTELNVGIDFNWEYPSSKHQHKKLVNRDLKTQSGSEMKKFLSTLTI
DGVTRSDQGLYTCAASSGLMTKKNSTFVRVHEKDKTHTCPPCPAPELLGGPSVFLFPP
KPKDTLMISRTPEVTCVVVDVSHEDPEVKFNWYVDGVEVHNAKTKPREEQYNSTYR
VVSVLTVLHQDWLNGKEYKCKVSNKALPAPIEKTISKAKGQPREPQVYTLPPSRDELT
KNQVSLTCLVKGFYPSDIAVEWESNGQPENNYKTTPPVLDSDGSFFLYSKLTVDKSRW
QQGNVFSCSVMHEALHNHYTQKSLSLSPGGGGGSGGGGSGGGGSGGGGSQVQLVES
GGGLVQPGGSLRLSCAASGSIFSFSTVRWYRQAPGKQREPVATLLDDGSTNYADSVKG
RFTISTDNAKNTVYLQMNSLKPEDTAVYYCNADGRTGWGPRGDYWGRGTQVTVSS
(SEQ ID NO: 31);

SDTGRPFVEMYSEIPEIIHMTEGRELVIPCRVTSPNITVTLKKFPLDTLIPDGK
RIIWDSRKGFIISNATYKEIGLLTCEATVNGHLYKTNYLTHRQTNTIIDVVLSPSHGIELS
VGEKLVLNCTARTELNVGIDFNWEYPSSKHQHKKLVNRDLKTQSGSEMKKFLSTLTI
DGVTRSDQGLYTCAASSGLMTKKNSTFVRVHEKDKTHTCPPCPAPELLGGPSVFLFPP
KPKDTLMISRTPEVTCVVVDVSHEDPEVKFNWYVDGVEVHNAKTKPREEQYNSTYR
VVSVLTVLHQDWLNGKEYKCKVSNKALPAPIEKTISKAKGQPREPQVYTLPPSRDELT
KNQVSLTCLVKGFYPSDIAVEWESNGQPENNYKTTPPVLDSDGSFFLYSKLTVDKSRW
QQGNVFSCSVMHEALHNHYTQKSLSLSPGGGGGSGGGGSGGGGSGGGGSQVQLVES
GGGLVQPGGSLRLSCAASGSSFKFSTVRWYRQAPGKQREPVATHDDGSTNYEDSVKG
RFTISRDNAKNTAFLQMNSLKPEDTAVYYCNADGSTGWGPRGDYWGRGTQVTVSS
(SEQ ID NO: 32).

In another aspect of the present disclosure, the present disclosure provides a nucleic acid molecule. According to an embodiment of the present disclosure, the nucleic acid molecule encodes the above-mentioned antibody or antigen-binding fragment, recombinant protein or multispecific antibody. The above-mentioned antibody or antigen-binding fragment, recombinant protein or multispecific antibody is encoded by the nucleic acid molecule according to an embodiment of the present disclosure.

According to an embodiment of the present disclosure, the nucleic acid molecule is DNA.

It should be noted that the nucleic acid molecule mentioned in the specification and claims of the present disclosure is understood by those skilled in the art to include either strand of a complementary double strand, or both strands. For convenience, only a single strand is provided in most cases in this specification and the claims; however, the complementary strand is also explicitly disclosed. Furthermore, the nucleic acid sequences disclosed herein include both DNA and RNA forms, and disclosure of one form is intended to also encompass the other.

In another aspect of the present disclosure, the present disclosure provides an expression vector. According to an embodiment of the present disclosure, the expression vector carries the above-mentioned nucleic acid molecule. When the above-mentioned nucleic acid molecule is inserted into the vector, it may be directly or indirectly linked to regulatory elements on the vector, as long as these regulatory elements are capable of controlling the translation and expression of the nucleic acid molecule. Such regulatory elements may be endogenous to the vector or exogenous, meaning they do not originate from the vector itself. The nucleic acid molecule is operably linked to the regulatory elements. The term "operably linked" refers to the insertion of a foreign gene into the vector in such a way that regulatory elements within the vector, such as transcriptional and translational control sequences, are capable of performing their intended function of regulating transcription and translation of the foreign gene. Commonly used vectors include, for example, plasmids and phage vectors. The expression vector according to some specific embodiments of the present disclosure, once introduced into suitable host cells, can efficiently express the aforementioned antibody or antigen-binding fragment, recombinant protein, or multispecific antibody under the control of an appropriate regulatory system, thereby enabling large-scale production of the antibody or antigen-binding fragment, recombinant protein, or multispecific antibody in vitro.

According to an embodiment of the present disclosure, the expression vector is a eukaryotic expression vector or prokaryotic expression vector, preferably a plasmid expression vector.

In another aspect of the present disclosure, the present disclosure provides a recombinant cell. According to an embodiment of the present disclosure, the recombinant cell includes the above-mentioned nucleic acid molecule or the above-mentioned expression vector; or the recombinant cell expresses the above-mentioned antibody or antigen-binding fragment, recombinant protein or multispecific antibody. Through the use of such recombinant cells under appropriate conditions, efficient expression of the above-mentioned antibody or antigen-binding fragment, recombinant protein, or multispecific antibody can be achieved within the cells.

It should be noted that the "appropriate conditions" mentioned in the specification of the present disclosure refers to the conditions suitable for expression of the antibody or antigen-binding fragment, recombinant protein, or multispecific antibody of the present disclosure. Those skilled in the art will readily appreciate that appropriate conditions for expression of the antibody or antigen-binding fragment, recombinant protein, or multispecific antibody include, but are not limited to, appropriate transformation or transfection methods, appropriate transformation or transfection conditions, a healthy physiological state of the host cells, appropriate host cell density, suitable culture conditions, and suitable culture duration. The term "appropriate conditions" is not particularly limited, and those skilled in the art can optimize the conditions based on the specific environment of the laboratory to achieve the optimal expression of the antibody or antigen-binding fragment, recombinant protein, or multispecific antibody.

According to an embodiment of the present disclosure, the recombinant cell is obtained by introducing the above-mentioned expression vector into a host cell.

According to an embodiment of the present disclosure, the recombinant cell is a eukaryotic cell.

According to an embodiment of the present disclosure, the recombinant cell is a mammalian cell.

In another aspect of the present disclosure, the present disclosure provides a pharmaceutical composition. According to an embodiment of the present disclosure, the pharmaceutical composition includes the above-mentioned antibody or antigen-binding fragment, recombinant protein, multispecific antibody, nucleic acid molecule, expression vector or recombinant cell. The pharmaceutical composition of the present disclosure is capable of binding to ANG-2 and blocks ANG-2 from binding to its respective receptors. Therefore, it can be effectively used in the treatment or prevention of angiogenesis-related diseases.

According to an embodiment of the present disclosure, the pharmaceutical composition further includes a pharmaceutically acceptable excipient.

In another aspect of the present disclosure, the present disclosure provides a conjugate. According to an embodiment of the present disclosure, the conjugate includes: the above-mentioned antibody or antigen-binding fragment, recombinant protein, or multispecific antibody; and a conjugation moiety linked to the above-mentioned antibody or antigen-binding fragment, recombinant protein, or multispecific antibody. The pharmaceutical composition of the present disclosure is capable of binding to ANG-2, and can be used to detect ANG-2 and also block ANG-2 from binding to its respective receptors. Therefore, it can be effectively used in the treatment or prevention of angiogenesis-related diseases.

According to an embodiment of the present disclosure, the conjugation moiety includes, but is not limited to, at least one of a carrier, drug, toxin, cytokine, protein tag, modifier, and chemotherapeutic agent.

The term "carrier" used herein may be a substance capable of suspending or dispersing in a liquid phase (e.g., particles, magnetic beads, and other solid-phase carriers), a solid-phase material capable of holding or accommodating a liquid phase (e.g., plates, membranes, test tubes, and other supports, as well as plate wells, microfluidic channels, glass capillaries, nanocolumns, monolithic columns, and other containers), or a labeling carrier for labeling an antibody or antigen-binding fragment, recombinant protein, or multispecific antibody, such as enzymes (e.g., peroxidase, alkaline phosphatase, luciferase, β-galactosidase); affinity substances (e.g., one of streptavidin and biotin, one strand of complementary sense and antisense nucleic acids); fluorescent substances (e.g., fluorescein, fluorescein isothiocyanate (FITC), rhodamine, green fluorescent protein (GFP), red fluorescent protein (RFP)); luminescent substances (e.g., luciferin, aequorin, acridinium esters, tris(2,2'-bipyridine)ruthenium, luminol); radioisotopes (e.g., ³H, ¹⁴C, ³²P, ³⁵S, ¹²⁵I); gold colloid and other labeling materials.

According to an embodiment of the present disclosure, the drug is a small molecule drug capable of binding to the antibody or antigen-binding fragment, recombinant protein or multispecific antibody, including but not limited to EGFR-TKI inhibitors, ALK inhibitors, verteporfin, and pegaptanib sodium.

According to an embodiment of the present disclosure, the toxin includes, but is not limited to, abrin, ricin A, Pseudomonas exotoxin, and diphtheria toxin.

According to an embodiment of the present disclosure, the cytokine includes, but is not limited to, IL-10, VEGF, EpCAM, GM2, and RANKL.

According to an embodiment of the present disclosure, the protein tag includes, but is not limited to, His tag, Flag tag, GST tag, MBP tag, SUMO tag, and C-Myc tag.

According to an embodiment of the present disclosure, the modifier should be interpreted broadly to refer to a substance used to modify a protein. Exemplarily, the modifier may be polyethylene glycol (PEG) or its derivatives.

According to an embodiment of the present disclosure, the chemotherapeutic agent includes, but is not limited to, albumin-bound paclitaxel, cyclophosphamide, ifosfamide, melphalan, methotrexate, fluorouracil, actinomycin D, and vincristine.

It should be noted that the method for conjugating the conjugation moiety with the antibody or antigen-binding fragment, recombinant protein, or multispecific antibody can be carried out using well-known methods in the art. For example, these methods include physical adsorption, covalent bonding, affinity-based approaches (e.g., biotin-streptavidin), and ionic bonding.

In another aspect of the present disclosure, the present disclosure provides a kit. According to an embodiment of the present disclosure, the kit includes the above-mentioned antibody or antigen-binding fragment, recombinant protein, multispecific antibody, nucleic acid molecule, expression vector, recombinant cell or conjugate. The antibody or antigen-binding fragment in the kit provided by the present disclosure can effectively bind to ANG-2. In addition, under appropriate conditions, the nucleic acid molecule, the expression vector, or the recombinant cell can express the antibody or antigen-binding fragment. Further, the kit containing the above-mentioned substances can effectively bind to ANG-2 and can be used to effectively detect ANG-2. The kit can be used for scientific research, such as qualitative or quantitative detection of ANG-2 in biological samples. The kit can also be used for assessment of an individual's condition, such as determining whether the ANG-2 level is higher or lower than normal after the ANG-2 level is obtained. The biological sample may be cells, tissues, or blood.

In another aspect of the present disclosure, the present disclosure provides use of the above-mentioned antibody or antigen-binding fragment, recombinant protein, multispecific antibody, nucleic acid molecule, expression vector, recombinant cell, or conjugate in the preparation of a kit for detecting ANG-2.

In another aspect of the present disclosure, the present disclosure provides use of the above-mentioned antibody or antigen-binding fragment, recombinant protein, multispecific antibody, nucleic acid molecule, expression vector, recombinant cell, pharmaceutical composition, or conjugate in the manufacture of a medicament for treating or preventing an angiogenesis-related disease.

According to an embodiment of the present disclosure, the angiogenesis-related disease includes at least one selected from cancer, ophthalmic disease, and inflammatory disease.

In another aspect of the present disclosure, the present disclosure provides use of the above-mentioned antibody or antigen-binding fragment, recombinant protein, multispecific antibody, nucleic acid molecule, expression vector, recombinant cell, pharmaceutical composition, or conjugate in the treatment or prevention of an angiogenesis-related disease.

According to an embodiment of the present disclosure, the angiogenesis-related disease includes at least one selected from cancer, ophthalmic disease, and inflammatory disease.

In another aspect of the present disclosure, the present disclosure provides use of the above-mentioned antibody or antigen-binding fragment, recombinant protein, multispecific antibody, nucleic acid molecule, expression vector, recombinant cell, pharmaceutical composition, or conjugate in the diagnosis, prognosis assessment, or staging of an angiogenesis-related disease.

According to an embodiment of the present disclosure, the angiogenesis-related disease includes at least one selected from cancer, ophthalmic disease, and inflammatory disease.

In another aspect of the present disclosure, the present disclosure provides a method for treating or preventing an angiogenesis-related disease. According to an embodiment of the present disclosure, the method includes: administering to a subject a pharmaceutically acceptable amount of the above-mentioned antibody or antigen-binding fragment, recombinant protein, multispecific antibody, pharmaceutical composition, or conjugate. The method of the present disclosure can effectively treat or prevent an angiogenesis-related disease.

The effective amount of the antibody or antigen-binding fragment, the recombinant protein, the multispecific antibody, the conjugate or the pharmaceutical composition of the present disclosure may vary with the mode of administration, the severity of the disease to be treated, and other factors. The preferred effective amount can be determined by a person skilled in the art based on various factors, for example, through clinical trials. These factors include, but are not limited to, pharmacokinetic parameters of the active ingredient, such as bioavailability, metabolism, and half-life; the severity of the patient's disease to be treated, patient's weight, patient's immune status, the route of administration. For example, depending on the exigencies of treatment conditions, several divided doses may be given per day, or the dosage may be reduced proportionally.

The antibody or antigen-binding fragment, recombinant protein, multispecific antibody, conjugate or pharmaceutical composition of the present disclosure may be incorporated into a drug suitable for parenteral administration (e.g., intravenous, subcutaneous, intraperitoneal, or intramuscular administration). These drugs may be formulated into a variety of forms, such as liquid, semi-solid, and solid dosage forms, including but not limited to, liquid solutions (e.g., injection solutions and infusion solutions) or lyophilized powders. Typical drugs are in the form of injection solutions or infusion solutions. The above-mentioned antibody or antigen-binding fragment, recombinant protein, multispecific antibody, pharmaceutical composition, or conjugate may be administered by intravenous infusion or injection, intramuscular injection, or subcutaneous injection.

According to an embodiment of the present disclosure, the route of administration in the method is subcutaneous injection or intravenous injection.

According to an embodiment of the present disclosure, the angiogenesis-related disease includes at least one selected from cancer, ophthalmic disease, and inflammatory disease.

In another aspect of the present disclosure, the present disclosure provides a method for diagnosing an angiogenesis-related disease. According to an embodiment of the present disclosure, the method includes: detecting ANG-2 in a test sample using the above-mentioned antibody or antigen-binding fragment, recombinant protein, multispecific antibody, nucleic acid molecule, expression vector, recombinant cell, kit, or conjugate; and determining the content of ANG-2 in the test sample based on a detection result.

According to an embodiment of the present disclosure, the method further includes: detecting VEGF in the test sample; and determining the content of VEGF in the test sample based on a detection result.

According to an embodiment of the present disclosure, the angiogenesis-related disease includes at least one selected from cancer, ophthalmic disease, and inflammatory disease.

In another aspect of the present disclosure, the present disclosure provides a method for assessing the prognosis of an angiogenesis-related disease. According to an embodiment of the present disclosure, the method includes: detecting ANG-2 in a test sample using the above-mentioned antibody or antigen-binding fragment, recombinant protein, multispecific antibody, nucleic acid molecule, expression vector, recombinant cell, kit, or conjugate; and determining the content of ANG-2 in the test sample based on a detection result.

According to an embodiment of the present disclosure, the method further includes: detecting VEGF in the test sample; and determining the content of VEGF in the test sample based on a detection result.

According to an embodiment of the present disclosure, the angiogenesis-related disease includes at least one selected from cancer, ophthalmic disease, and inflammatory disease.

In another aspect of the present disclosure, the present disclosure provides a method for staging an angiogenesis-related disease. According to an embodiment of the present disclosure, the method includes: detecting ANG-2 in a test sample using the above-mentioned antibody or antigen-binding fragment, recombinant protein, multispecific antibody, nucleic acid molecule, expression vector, recombinant cell, kit, or conjugate; and determining the content of ANG-2 in the test sample based on a detection result.

According to an embodiment of the present disclosure, the method further includes: detecting VEGF in the test sample; and determining the content of VEGF in the test sample based on a detection result.

According to an embodiment of the present disclosure, the content of ANG-2 and/or VEGF in the test sample not lower than a minimum level for a stage IV tumor indicates that the test sample is derived from a patient with a stage IV tumor;
the content of ANG-2 and/or VEGF in the test sample falling between a level for a stage **III** tumor and a level for a stage IV tumor indicates that the test sample is derived from a patient with a stage **III** tumor;
the content of ANG-2 and/or VEGF in the test sample falling between a level for a stage II tumor and a level for a stage **III** tumor indicates that the test sample is derived from a patient with a stage II tumor;
the content of ANG-2 and/or VEGF in the test sample falling between a level for a stage I tumor and a level for a stage II tumor indicates that the test sample is derived from a patient with a stage I tumor.

According to an embodiment of the present disclosure, the angiogenesis-related disease includes at least one selected from cancer, ophthalmic disease, and inflammatory disease.

In another aspect of the present disclosure, the present disclosure provides the above-mentioned antibody or antigen-binding fragment, recombinant protein, multispecific antibody, pharmaceutical composition, or conjugate for use in the treatment or prevention of an angiogenesis-related disease.

According to an embodiment of the present disclosure, the angiogenesis-related disease includes at least one selected from cancer, ophthalmic disease, and inflammatory disease.

According to another aspect of the present disclosure, the present disclosure provides the above-mentioned antibody or antigen-binding fragment, recombinant protein, multispecific antibody, nucleic acid molecule, expression vector, recombinant cell, kit, or conjugate for use in the diagnosis, prognosis assessment, or staging of an angiogenesis-related disease.

According to an embodiment of the present disclosure, the angiogenesis-related disease includes at least one selected from cancer, ophthalmic disease, and inflammatory disease.

### Beneficial Effects:

1. The ANG-2 single-domain antibody variable region obtained by screening in the present disclosure exhibits high binding activity to ANG-2, and can effectively block the binding of ANG-2 to its receptor TIE2, thereby effectively treating or preventing angiogenesis-related diseases.
2. The bispecific inhibitor prepared by using the ANG-2 single-domain antibody variable region and the VEGF receptor-Fc fusion protein in the present disclosure can simultaneously bind to VEGF and ANG-2, and exerts anti-angiogenesis effects and reduces vascular inflammations by blocking the binding of VEGF to VEGFR and the binding of ANG-2 to TIE2. Therefore, it can be effectively used in the treatment or prevention of angiogenesis-related diseases. Furthermore, the bispecific inhibitor of the present disclosure demonstrates significantly superior blocking activity against both VEGF and ANG-2 compared to the marketed drug Faricimab.

Additional aspects and advantages of the present disclosure will be partially given in the following description, partially become obvious from the following description, or be learned through the practice of the present disclosure.

### BRIEF DESCRIPTION OF THE DRAWINGS

The above and/or additional aspects and advantages of the present disclosure will become obvious and easy to understand from the description of the embodiments in conjunction with the following accompanying drawings.
FIG. 1 shows the affinity detection results of the ANG-2 single-domain antibody in Example 3 of the present disclosure.
FIG. 2 is a structural schematic diagram of a bispecific inhibitor in Example 4 of the present disclosure.
FIG. 3 shows the VEGF reporter gene activity detection results of the bispecific inhibitor in Example 6 of the present disclosure.
FIG. 4 shows the HUVEC proliferation activity detection results of the bispecific inhibitor in Example 6 of the present disclosure.
FIG. 5 shows the ANG-2 blocking activity assay results of the bispecific inhibitor in Example 7 of the present disclosure.

### DETAILED DESCRIPTION

The embodiments of the present disclosure are described in detail below. The embodiments described below are exemplary, and are only intended to explain the present disclosure, rather than being construed as limitations to the present disclosure.

It should be noted that the terms "first" and "second" are used only for the purpose of description and should not be construed as indicating or implying relative importance or implicitly indicating the number of technical features indicated. Thus, the features defined by the terms "first" and "second" may include one or more of the features either explicitly or implicitly. Further, in the description of the present disclosure, unless otherwise stated, the term "multiple" or "more" means two or more.

The term "comprising" or "including" is an open-ended expression, that is, including the content indicated in the present disclosure, but not excluding other aspects.

The term "optionally", "optional" or "option" means that the subsequent event or condition may but may not occur necessarily, and this description includes a circumstance in which the event or condition occurs and a circumstance in which no event or condition occurs.

The term "antibody" generally refers to an antibody that can recognize one or more antigen epitopes, including but not limited to, monoclonal antibodies, polyclonal antibodies, multimeric antibodies, and CDR-grafted antibodies.

The term "monoclonal antibody" refers to an antibody that recognizes a single specific antigen epitope. A typical monoclonal antibody includes two light chains (L chains) with lower molecular weight and two heavy chains (H chains) with higher molecular weight, which are linked by disulfide bonds to form a four-chain structure. The amino-terminal (N-terminal) amino acid sequence of the heavy or light chain exhibits significant variation and is referred to as the variable region (V region), while the carboxyl-terminal (C-terminal) is relatively stable with minimal variation and is called the constant region (C region). The variable regions of the light chain and heavy chain are designated as VL and VH, respectively.

The N-terminal of the heavy or light chain contains a variable region (V region), while the C-terminal contains a relatively constant region (C region). The light chain and heavy chain variable regions are denoted as VL and VH, respectively. Monoclonal antibodies can also exist as small-molecule antibodies, which mainly include Fab antibodies, Fv antibodies, single-chain antibodies, single-domain antibodies, and minimal recognition units.

The term "Fab antibody" generally refers to an antibody containing only the Fab region, composed of heavy chain VH and CH1 and a complete light chain, with the light chain and heavy chain connected by a disulfide bond.

The term "Fv antibody" generally refers to an antibody composed of only the variable region of the light chain (VL) and the variable region of the heavy chain (VH), connected via a non-covalent bond. This is the smallest functional fragment that retains a complete antigen-binding site.

The terms "single-domain antibody", "nanobody", and "VHH antibody" are used interchangeably, and initially described as an antigen-binding immunoglobulin (variable) domain of "heavy-chain antibodies" (i.e., "antibodies devoid of light chains") (Hamers-Casterman C, Atarhouch T, Muyldermans S, Robinson G, Hamers C, Songa EB, Bendahman N, Hamers R.: "Naturally occurring antibodies devoid of light chains"; Nature 363, 446-448 (1993)). These antibodies only contain the heavy chain variable (VH) region along with conventional CH2 and CH3 domains, and they bind specifically to antigens via the heavy chain variable region.

The term "single-chain antibody" generally refers to an antibody where a heavy chain variable region (VH) and a light chain variable region (VL) are linked via a linking peptide.

The terms "minimal recognition unit" and "MRU" both refer to an antibody composed of only one CDR, which has a very small molecular weight of about 1% of a complete antibody.

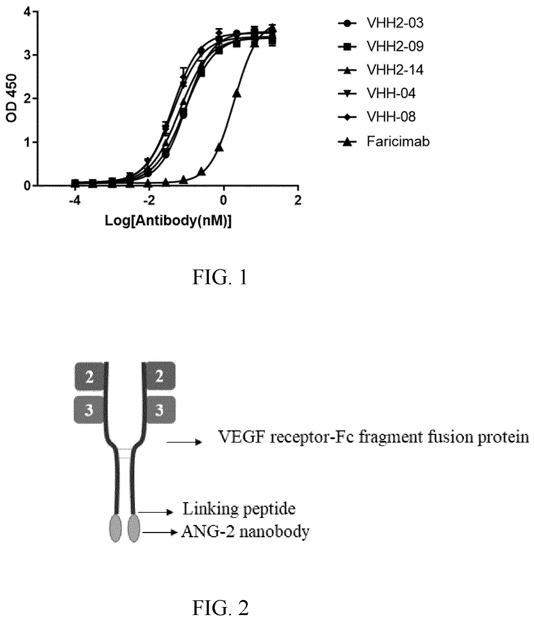

The term "multispecific antibody" herein refers to an antibody capable of recognizing multiple antigen epitopes, such as bispecific antibody capable of recognizing two antigen epitopes (abbreviated as bsAb), trispecific antibody capable of recognizing three antigen epitopes, or tetraspecific antibody capable of recognizing four antigen epitopes. It is understood in broad sense. The specific structures are not limited as long as they can recognize multiple antigen epitopes. Exemplarily, the multispecific antibody is the bispecific antibody as shown in FIG. 1.

The terms "CDR-grafted antibody" and "modified antibody" both refer to an antibody where the CDRs of a monoclonal antibody from one species are grafted onto the variable region of an antibody from another species. For example, the CDRs of a murine monoclonal antibody can be grafted onto a human antibody to replace the CDRs of the human antibody, thereby retaining the antigen specificity of the murine antibody while reducing immunogenicity. It should be noted that in the present disclosure, both the multispecific antibody and the monoclonal antibody can be CDR-grafted antibodies.

The terms "symmetric bispecific antibody" and "symmetric bsAb" are synonymous and generally refers to an antibody with two or more identical peptide chains, the number of which is not specifically limited. For example, for a bispecific antibody containing an Fc fragment, the Fc regions of the bispecific antibody are positioned on separate polypeptide chains, and a symmetric BsAb refers to a bispecific antibody with two peptide chains having identical structures and sequences. Exemplarily, in the present disclosure, the symmetric BsAb has two identical polypeptide chains, each containing a first binding region, a linker peptide, and a second binding region. Specifically, the first binding region includes an antibody or antigen-binding fragment capable of recognizing ANG-2; the second binding region includes immunoglobulin-like domain 2 of VEGFR1 fragment, immunoglobulin-like domain 3 of VEGFR2, and an Fc fragment; the C-terminus of immunoglobulin-like domain 2 of the VEGFR1 fragment is linked to the N-terminus of immunoglobulin-like domain 3 of VEGFR2; the C-terminus of immunoglobulin-like domain 3 of the VEGFR2 fragment is linked to the N-terminus of the Fc fragment; the C-terminus of the Fc fragment is linked to the N-terminus of the linking peptide; the C-terminus of the linking peptide is linked to the N-terminus of the antibody or antigen-binding fragment. This bispecific antibody is illustrated in FIG. 1 for details.

The terms "asymmetric bispecific antibody" and "asymmetric bsAb" are synonymous and generally refers to an antibody with two or more different peptide chains, the number of which is not specifically limited. For example, for a bispecific antibody containing an Fc fragment, the Fc regions of the bispecific antibody are positioned on separate polypeptide chains, and an asymmetric BsAb refers to a bispecific antibody with two peptide chains having different structures or sequences. Exemplarily, the two peptide chains of the Fc fragment of the bispecific antibody are respectively linked to two different binding regions (i.e., the first binding region and the second binding region). Specifically, the first binding region includes an antibody or antigen-binding fragment capable of recognizing ANG-2; the second binding region includes immunoglobulin-like domain 2 of VEGFR1 fragment and immunoglobulin-like domain 3 of VEGFR2; the C-terminus of immunoglobulin-like domain 2 of the VEGFR1 fragment is linked to the N-terminus of immunoglobulin-like domain 3 of VEGFR2; the N-terminus of one peptide chain of the Fc fragment is linked to the C-terminus of immunoglobulin-like domain 3 of the VEGFR2 fragment; the N-terminus of the other peptide chain is linked to the C-terminus of the antibody or antigen-binding fragment.

Further, two peptide chains of the Fc fragment of the asymmetric antibody may be connected via a "knob-into-hole" (KiH)" structure, which involves the formation of knob-hole mutations in the CH3 domain of the constant region of the antibody heavy chain, facilitating the occlusion of the heavy chain and heterodimer formation. For example, it is achieved by mutating amino acids (T366S, L368A, Y407V, and Y349C mutations in one chain, representing "hole"; in the other chain, T366W and S354C mutations, representing "knob") in the CH3 domain of the constant region of human IgG1 heavy chain.

The term "fragment" refers to a target protein or polypeptide, as well as a target protein or polypeptide that has a truncated N-terminal (N-end) or C-terminal (C-end), and/or internal deletions. For example, a VEGF receptor fragment can refer to a full-length VEGF receptor (VEGFR1, VEGFR2, or VEGFR3) or a truncated VEGF receptor (VEGFR1, VEGFR2, or VEGFR3). The polypeptide may be. Exemplarily, a VEGF receptor fragment is immunoglobulin-like domain 2 of VEGFR1 and/or immunoglobulin-like domain 3 of VEGFR2.

The term "identity", "homology", or "similarity" is used to describe the percentage of identical amino acids or nucleotides between two amino acid sequences or nucleic acid sequences relative to a reference sequence, determined using conventional methods (see, for example, Ausubel et al., eds. (1995), Current Protocols in Molecular Biology, Chapter 19 (Greene Publishing and Wiley-Interscience, New York); and ALIGN (Dayhoff (1978), Atlas of Protein Sequence and Structure 5: Suppl. 3 (National Biomedical Research Foundation, Washington, D.C.). There are many algorithms for aligning sequences and determining sequence identity, including: a homology alignment algorithm of Needleman et al. (1970) J.Mol.Biol.48:443; a local homology algorithm of Smith et al. (1981) Adv. Appl. Math. 2:482; a similarity search method of Pearson et al. (1988) Proc.Natl.Acad.Sci.85:2444; the Smith-Waterman algorithm (Meth. Mol. Biol. 70: 173-187(1997); as well as BLASTP, BLASTN, and BLASTX algorithms (see Altschul et al. (1990) J. Mol. Biol. 215:403-410). Computer programs that utilize these algorithms are also available, including but not limited to, ALIGN or Megalign (DNASTAR) software, WU-BLAST-2 (Altschul et al., Meth. Enzym., 266:460-480 (1996)); or GAP, BESTFIT, BLAST (Altschul et al., ditto), FASTA, and TFASTA (available in the Genetics Computing Group (GCG) package, 8th edition, Madison, Wisconsin, USA); and CLUSTAL (in PC/Gene programs provided by Intelligenetics, Mountain View, California).

As used herein, the term "at least 80% identity" means at least 80% identity with each reference sequence, which may be 80%, 81%, 82%, 83%, 84%, 85%, 86%, 87%, 88%, 89%, 90%, 91%, 92%, 93%, 94%, 95%, 96%, 97%, 98%, 99%, 99.5%, or 99.9%.

The term "expression vector" generally refers to a nucleic acid molecule capable of being inserted into a suitable host and self-replication. It transfers an inserted nucleic acid sequence into host cells and/or between host cells. The expression vector may include a vector that is primarily used for inserting DNA or RNA into cells, a vector that is primarily used for replicating DNA or RNA, and a vector for expression that is primarily used for transcription and/or translation of DNA or RNA. The expression vector further includes a vector with multiple above-mentioned functions. The expression vector may be a polynucleotide capable of being transcribed and translated into a polypeptide when being introduced into suitable host cells. Typically, by culturing the suitable host cells containing the expression vector, the expression vector can produce a desired expression product.

The term "recombinant cell" generally refers to a cell that has undergone modification, engineering, or recombination of the genetic substance of the host cell using a genetic engineering technology or cell fusion technology to obtain a stably-inherited unique trait. The term "host cell" refers to a prokaryotic or eukaryotic cell into which a recombinant expression vector can be introduced. The term "transformed" or "transfected" refers to the introduction of nucleic acids (e.g., vectors) into cells by various technologies known in the art. Suitable host cells can be transformed or transfected with DNA sequences in the present disclosure and may be used for the expression and/or secretion of target proteins. Examples of suitable host cells that may be used in the present disclosure include immortalized hybridoma cells, NS/0 myeloma cells, 293 cells, Chinese hamster ovary (CHO) cells, HeLa cells, Cap cells (human amniotic fluid-derived cells), and CoS cells.

The term "pharmaceutical composition" generally refers to a unit dosage form that can be prepared by means of any of the methods well-known in the pharmaceutical field. All methods include a step of combining an active ingredient with a carrier that make up one or more of accessory components. Typically, a composition is prepared by uniformly and thoroughly mixing an active compound with a liquid carrier, a finely ground solid carrier, or both.

The term "pharmaceutically acceptable excipient" may include any solvent, solid excipient, diluent, or other liquid excipient suitable for the specific dosage form. Apart from any range of conventional excipients that are incompatible with the compound of the present disclosure, such as any adverse biological effects produced or any harmful interactions of any other components of a pharmaceutically acceptable composition, their uses are also within the scope considered by the present disclosure.

The term "administration" refers to the introduction of a predetermined amount of a substance into a patient in a suitable manner. The antibody or antigen-binding fragment, the recombinant protein, the multispecific antibody, the conjugate, or the pharmaceutical composition of the present disclosure can be administered through any common route, as long as they reach the intended tissue. Various modes of administration are expected, including intraperitoneal injection, intravenous injection, intramuscular injection, or subcutaneous injection, but the present disclosure is not limited to these given examples of modes of administration. Preferably, the composition of the present disclosure is administered by intravenous injection or subcutaneous injection.

The term "treatment" herein refers to achieving a desired pharmacological and/or physiological effect. The effect may be prophylactic in terms of complete or partial prevention of diseases or their symptoms, and/or may be therapeutic in terms of partial or complete cure of disease and/or adverse effects caused by the diseases. The term "treatment" as used herein encompasses diseases of mammals, particularly humans, including: (a) prevention of a disease or condition in individuals who are susceptible to disease but have not yet been diagnosed definitely; (b) inhibition of a disease, e.g., arresting the development of the disease; or (c) alleviation of a disease, e.g., mitigating symptoms associated with the disease. The "treatment" as used herein encompasses any medication in which a medicament or compound is administered to an individual to treat, cure, alleviate, improve, mitigate, or suppress an individual's disease, including, but not limited to, administering a drug containing the compound described herein to an individual in need.

The term "an angiogenesis-related disease" herein generally refers to diseases caused by angiogenesis (or referred to as neovascularization), including, but not limited to, cancer, ophthalmic diseases, and inflammatory diseases. Exemplarily, the cancer may be solid tumors or hematologic tumors, including but not limited to, hemangiomas, gastric cancer, liver cancer, lung cancer, breast cancer, colon cancer, nasopharyngeal cancer, bladder cancer, cervical cancer, prostate cancer, bone cancer, skin cancer, thyroid cancer, kidney cancer, esophageal cancer, melanoma, fibrosarcoma, rhabdomyosarcoma, astrocytoma, neuroblastoma, and glioma; the ophthalmologic diseases may be intraocular neovascular syndromes (e.g., proliferative retinopathies, age-related macular degeneration, AMD); the inflammatory diseases include, but are not limited to, rheumatoid arthritis (RA) and psoriasis.

The embodiments of the present disclosure will be illustrated below in conjunction with the examples. Those skilled in the art will understand that the following examples are only used to illustrate the present disclosure, but not to limit the scope of the present disclosure. If specific techniques or conditions are not explicitly stated in the examples, they should be interpreted in accordance with the techniques or conditions described in the literature in the art or in accordance with the product manuals. The reagents and instruments used without indicating the manufacturers are all conventional products that can be purchased commercially.

In the examples of the present disclosure, the control drug used is Faricimab, purchased from Shanghai Biointron Biotech Co., Ltd., with the catalog number B936701.

### Example 1: Screening of ANG-2 Single-Domain Antibodies (Referred to as Single-Domain Antibodies)

### 1. Construction of Phage Display Library

Two alpacas were immunized five times with human ANG-2 protein (purchased from Sino Biological, catalog number: 10691-H08H). Peripheral blood (50 mL per alpaca) was collected after the fifth immunization. Lymphocytes were isolated using a lymphocyte separation solution, and total RNA was extracted using the RNAiso Plus reagent. The extracted RNA was then reverse-transcribed into cDNA using the PrimeScript II kit (TAKARA 6210A). The target VHH gene fragment, corresponding to the variable region of the single-domain antibody, was amplified using nested PCR.

The amplified VHH gene was digested with Sfi I, ligated into the pComb3xss vector using T4 DNA ligase, and then electroporated into competent TG1 cells to construct the VHH gene library, and a transformed culture was obtained. Aliquots of 0.1 µL, 0.01 µL, 0.001 µL, and 0.0001 µL of the transformed culture were spread on 90 mm agar plates, and the total library capacity was calculated to be 4.75×10⁹ cfu. 100 randomly selected colonies were amplified and sequenced, confirming a 100% insertion rate.

A culture containing 10-100 times the library capacity of living cells was inoculated, cultured to the logarithmic phase, and then rescued using M13K07 phage. Afterward, phages were collected by centrifugation and purified using PEG-NaCl, thereby obtaining a phage display library, with a titer of 6.8×10¹³ cfu/mL. The phage display library was directly used for subsequent affinity screening of specific phages.

### 2. Screening of the Phage Display Library

Human ANG-2 protein (Sino Biological, catalog number: 10691-H08H) was used to coat plates at two concentrations: 100 µg/mL and 50 µg/mL, incubated overnight at 4°C, and then screened separately. The next day, the plates were blocked with 1% ovalbumin (OVA) at 37°C for 1 hour. 100 µL of phage (6.8×10¹³ cfu/mL) was added and incubated. Unbound phages were removed by washing five times with PBST (PBS containing 0.05% Tween-20). Phages specifically bound to human ANG-2 protein were eluted with 100 µL of 0.1 M Gly-HCl (pH 2.2) and used to infect log-phase E. coli TG1 cells. The phages were amplified and purified for the next round of selection. The selection procedure was repeated for two additional rounds. After the final round, the obtained phages were used to infect E. coli TG1, and the resulting colonies were plated. Single clones were picked from the plates for sequencing. The protein sequences of each clone were analyzed according to the sequence alignment results, and clones with different CDR1, CDR2, and CDR3 sequences were regarded as different antibody strains. Finally, a total of 5 different single-domain antibodies were obtained. The heavy chain variable regions (VHH) of the 5 single-domain antibodies were VHH-04 with an amino acid sequence specified in SEQ ID NO: 16, VHH-08 with an amino acid sequence specified in SEQ ID NO: 17, VHH2-03 with an amino acid sequence specified in SEQ ID NO: 18, VHH2-09 with an amino acid sequence specified in SEQ ID NO: 19, and VHH2-14 with an amino acid sequence specified in SEQ ID NO: 20.

### Example 2: Preparation of ANG-2 Single-Domain Antibody

The nucleotide sequences of the variable regions of the five single-domain antibody clones obtained in Example 1 (SEQ ID NOs: 33-37) were linked to the nucleotide sequence of the human IgG1 Fc region (SEQ ID NO: 39). The specific sequences are listed in Table 1. These sequences were synthesized, inserted into the pcDNA3.1 expression plasmid, and then transformed into E. coli, followed by plasmid extraction. The expression plasmids were filtered through a 0.22 µm membrane and subsequently transfected into CHO cells using the PEI transfection reagent (Polysciences, 08APR22). After four days of culture, the cell culture supernatant was collected, added into a fully suspended Protein A resin solution, mixed well, and incubated at 4°C for 1 hour. The Protein A-supernatant mixture was then transferred into a gravity column, and once the liquid had flowed through completely, the Protein A resin was washed with 10 column volumes of elution buffer. The eluted protein fraction was collected and immediately neutralized by adding 4 mL of 1M Tris-HCl buffer (pH 8.0), followed by incubation at room temperature for 5 minutes. The target products, ANG-2 single-domain antibodies, were obtained and designated as VHH-04, VHH-08, VHH2-03, VHH2-09 and VHH2-14 antibodies.

**Table 1: Sequences of VHH antibodies**

| Name | | Single-Domain Antibody Variable Region | Human IgG1 Fc | Antibody Amino Acid Sequence |
|---|---|---|---|---|
| VHH-04 Antibody | Amino Acid Sequence | SEQ ID NO: 16 | SEQ ID NO: 38 | SEQ ID NO: 21 |
| | Nucleotide Sequence | SEQ ID NO: 33 | SEQ ID NO: 39 | |
| VHH-08 Antibody | Amino Acid Sequence | SEQ ID NO: 17 | SEQ ID NO: 38 | SEQ ID NO: 22 |
| | Nucleotide Sequence | SEQ ID NO: 34 | SEQ ID NO: 39 | |
| VHH2-03 Antibody | Amino Acid Sequence | SEQ ID NO: 18 | SEQ ID NO: 38 | SEQ ID NO: 23 |
| | Nucleotide Sequence | SEQ ID NO: 35 | SEQ ID NO: 39 | |
| VHH2-09 Antibody | Amino Acid Sequence | SEQ ID NO: 19 | SEQ ID NO: 38 | SEQ ID NO: 24 |
| | Nucleotide Sequence | SEQ ID NO: 36 | SEQ ID NO: 39 | |
| VHH2-14 Antibody | Amino Acid Sequence | SEQ ID NO: 20 | SEQ ID NO: 38 | SEQ ID NO: 25 |
| | Nucleotide Sequence | SEQ ID NO: 37 | SEQ ID NO: 39 | |

CAGCTGCAGCTGGTGGAGTCCGGAGGAGGACTGGTGCAGCCTGGAGG
AAGCCTGAGGCTGTCCTGCGCTGCTTCCGGATTTACCCTGGGATATTACTCTATTGG
CTGGTTCAGGCAGGCCCCCGGAAAAGAGAGAGAGGGAGTGTCTTGCATTTCCCGC
ACAGACGGAAGCACCTACTACGCTGACTCCGTCAAGGGCAGGTTCACAATCTCCC
GCGACAATGCCAAGAACACAGTGTACCTGCAGATGAACAACCTGAAGCCCGAGG
ACACCGCCGTGTATTACTGCGCTGTGAAGGGCGCTAGGTATAGCGGAAGCTACCTG
TGCGGCCTGGACGAGTATGACTACTGGGGACAGGGCACCCAGGTCACCGTGTCCT
CC (SEQ ID NO: 33);

CAGGTGCAGCTGGTGGAGTCCGGAGGAGGACTGGTGCAGCCTGGAGG
AAGCCTGAGGCTGTCCTGCGCTGCTTCCGGATTTAGTTTTAGTAGTGCAACAATGT
CCTGGCACAGGCAGACCCCTGGAAAAGAGAGGACAATGGTTGCTCTGATTGCCTA
CGCCGGAGGATCTACAACCTACGCTGATTCAGTGAAGGGCCGGTTCACCATCTCCA
GAGACAACGCTAAGAACACCGTGTACCTGCAGATGAGCAACCTGAAGCCTGAAG
ATACTGCTGTGTATTACTGCAACACCAGGAGGTACAACAGGGACTTTTGGGGCCAG
GGCACCCAGGTGACCGTGTCCTCC (SEQ ID NO: 34);

CAGCTGCAGCTGGTGGAGTCCGGAGGAGGACTGGTGCAGCCTGGAGG
AAGCCTGAGGCTGTCCTGCGCTGCTTCCGGATTTACCCTGGACTATTATGCCATTGG
CTGGTTCCGGCAGGTCCCTGGAAAAGCTAGAGAGGGACTGAGCTGCATTTCCTCT
GGAGACGGGTCCACCTACTACGCTGATTCTGTGAAGGGCAGATTTACCATCTCCCG
GGACAATACTAAGAACACCGTGTACCTGCAGATGAACAGCCTGAAACCCGAGGAC
ACCGCCGTGTATTCCTGCGCTGCTGTGAGATCCGGCTCTAAGTGTCTGGTGACCAG
GGGTACCGTGGTGGCTGCTGGACAGCTGTATGAATACTGGGGCCAGGGCACACAG
GTGACAGTGTCCTCC (SEQ ID NO: 35);

CAGGTGCAGCTGGTGGAGTCCGGAGGAGGACTGGTGCAGCCTGGAGG
AAGCCTGAGGCTGTCCTGCGCTGCTTCCGGAAGCATTTTTAGTTTTAGTACCGTGA
GGTGGTACAGGCAGGCTCCTGGAAAACAGAGGGAGCCTGTGGCTACTCTGCTGGA
TGACGGTTCCACCAACTACGCCGACTCCGTGAAGGGAAGGTTCACCATCTCCACC
GACAACGCCAAGAACACAGTCTATCTGCAGATGAACAGCCTGAAACCCGAAGATA

CTGCTGTGTACTACTGCAACGCCGACGGAAGGACCGGATGGGGACCTAGGGGAGA
CTATTGGGGAAGGGGAACCCAGGTGACCGTGTCCTCC (SEQ ID NO: 36);

CAGGTGCAGCTGGTGGAGTCCGGAGGAGGACTGGTGCAGCCTGGAGG
AAGCCTGAGGCTGTCCTGCGCTGCTTCCGGAAGCTCCTTTAAATTTTCCACAGTCA
GGTGGTACAGGCAGGCCCCTGGAAAACAGAGGGAGCCTGTGGCTACTCATGACG
ACGGATCTACCAACTACGAGGACTCCGTGAAAGGCAGGTTCACAATCAGCAGGGA
CAACGCCAAGAACACCGCATTCCTGCAGATGAACAGCCTGAAGCCTGAGGATACC
GCCGTGTACTACTGCAACGCCGACGGATCTACCGGGTGGGGACCTAGGGGAGACT
ATTGGGGAAGGGGAACCCAGGTGACCGTCTCCTCC (SEQ ID NO: 37);

EPKSADKTHTCPPCPAPELLGGPSVFLFPPKPKDTLMISRTPEVTCVVVDVS
HEDPEVKFNWYVDGVEVHNAKTKPREEQYNSTYRVVSVLTVLHQDWLNGKEYKCK
VSNKALPAPIEKTISKAKGQPREPQVYTLPPSREEMTKNQVSLTCLVKGFYPSDIAVEW
ESNGQPENNYKTTPPVLDSDGSFFLYSKLTVDKSRWQQGNVFSCSVMHEALHNHYT
QKSLSLSPGK (SEQ ID NO: 38);

GAGCCCAAATCTGCTGACAAAACTCACACATGCCCACCGTGCCCAGCA
CCTGAACTCCTGGGGGGACCGTCAGTCTTCCTCTTCCCCCCAAAACCCAAGGACA
CCCTCATGATCTCCCGGACCCCCGAGGTCACATGCGTGGTGGTGGACGTGAGCCA
CGAAGACCCTGAGGTCAAGTTCAACTGGTACGTGGACGGCGTGGAGGTGCATAAT
GCCAAGACAAAGCCGCGGGAGGAGCAGTACAACAGCACGTACCGTGTGGTCAGC
GTCCTCACCGTCCTGCACCAGGACTGGCTGAATGGCAAGGAGTACAAGTGCAAGG
TCTCCAACAAAGCCCTCCCAGCCCCCATCGAGAAAACCATCTCCAAAGCCAAAGG
GCAGCCCCGAGAACCACAGGTGTACACCCTGCCCCCATCCCGGGAGGAGATGACC
AAGAACCAGGTCAGCCTGACCTGCCTGGTCAAAGGCTTCTATCCCAGCGACATCG
CCGTGGAGTGGGAGAGCAATGGGCAGCCGGAGAACAACTACAAGACCACGCCTC
CCGTGCTGGACTCCGACGGCTCCTTCTTCCTCTACAGCAAGCTCACCGTGGACAA
GAGCAGGTGGCAGCAGGGGAACGTCTTCTCATGCTCCGTGATGCATGAGGCTCTG
CACAACCACTACACGCAGAAGAGCCTCTCCCTGTCTCCGGGTAAA (SEQ ID NO: 39).

### Example 3: Affinity Assay for ANG-2 Single-Domain Antibody

Human ANG-2 protein (Sino Biological, 10691-H08H) was diluted to prepare a 1 µg/mL coating solution, which was added to an ELISA plate at 100 µL/well and coated at 2-8°C for more than 12 h. The residual coating solution was discarded, and 200 µL of 2% BSA-PBS was added per well for blocking at 37°C for 2 hours, followed by three washes with 300 µL of PBST per well. The test samples (VHH-04, VHH-08, VHH2-03, VHH2-09, and VHH2-14 antibody prepared in Example 2) and the control drug (Faricimab, Biointron Biotech, Cat. No. B936701, Lot No. 20220108Z003) were diluted to 20 nM in PBS, then serially diluted in a three-fold gradient across eight concentrations, subsequently added to the ELISA plate at 100 µL/well, and incubated at 37°C for 1 hour, followed by three washes with 300 µL of PBST per well. Goat anti-Human Fc-HRP antibody (Abcam, ab97225) diluted in 1: 10,000 was added at 100 µL/well, and incubated at 37°C for 1 hour, followed by three washes with 300 µL of PBST per well. 100 µL of TMB substrate solution was added per well and incubated at room temperature for 5 minutes. The reaction was stopped with 50 µL of 2M H₂SO₄ per well. The ELISA plate was then placed on a microplate reader, and the absorbance at 450 nm (OD₄₅₀) was read. GraphPad Prism 7.0 software was used to analyze data for fitting of the binding activity to obtain an EC₅₀ value of ANG-2-binding activity. The results are shown in Table 2 and FIG. 1. The results showed that VHH-04, VHH-08, VHH2-03, VHH2-09 and VHH2-14 antibodies exhibited significant binding activity to human ANG-2, with superior binding activity compared to Faricimab.

**Table 2: ANG-2 Binding Activity of Different Single-Domain Antibodies**

| Sample | EC₅₀ (nM) | Relative activity |
|---|---|---|
| VHH-04 | 0.044 | 4250% |
| VHH-08 | 0.042 | 4450% |
| VHH2-03 | 0.096 | 1950% |
| VHH2-09 | 0.088 | 2130% |
| VHH2-14 | 0.065 | 2880% |
| Faricimab | 1.871 | 100% |

### Example 4: Preparation of Tetravalent Bispecific Inhibitor (Also Known as Bispecific Antibody or BsAb)

The VEGF receptor-Fc fusion protein with a nucleotide sequence specified in SEQ ID NO: 15 and the ANG-2 single-domain antibody variable region with a nucleotide sequence specified in any one of SEQ ID NOs: 33-37 were linked via a linking peptide with a nucleotide sequence specified in SEQ ID NO: 40, then synthesized and cloned into the expression plasmid pcDNA3.1, and transformed into E. coli, followed by plasmid extraction. The expression plasmid was filtered through a 0.22 µm filter membrane and transfected into HEK 293 cells using PEI transfection reagent (Polysciences, 08APR22). After 20-24 hours, Feed (Sino Biological, M293-SUPI) was added and then supplied every other day for a total of 6 days of culture. On Day 6, the cell culture supernatant was collected. Fully resuspended Protein A resin was added, mixed well, and incubated at 4°C for 1 hour. The mixture of Protein A and cell culture supernatant was transferred to a gravity column. After the liquid in the mixture flowed through completely, Protein A gel resin washed with 10 times the column volume of elution buffer, and the protein eluate was collected. 4 mL of 1M Tris-HCl neutralization solution (pH 8.0) was added and incubated at room temperature for 5 min to obtain target products, i.e., tetravalent bispecific inhibitors, designated as GB10-04, GB10-08, GB10-03, GB10-09 and GB10-14 antibodies. The specific sequences are shown in Table 3, and their structural representation is shown in FIG. 2.

**Table 3: Sequences of Tetravalent Bispecific Inhibitors**

| Name | | VEGF Receptor-Fc Fusion Protein | Linking Peptide | Single-Domain Antibody Variable Region | Bispecific Inhibitor Amino Acid Sequence |
|---|---|---|---|---|---|
| GB10-04 | Amino Acid Sequence | SEQ ID NO: 26 | SEQ ID NO: 27 | SEQ ID NO: 16 | SEQ ID NO: 28 |
| | Nucleotide Sequence | SEQ ID NO: 15 | SEQ ID NO: 40 | SEQ ID NO: 33 | |
| GB10-08 | Amino Acid Sequence | SEQ ID NO: 26 | SEQ ID NO: 27 | SEQ ID NO: 17 | SEQ ID NO: 29 |
| | Nucleotide Sequence | SEQ ID NO: 15 | SEQ ID NO: 40 | SEQ ID NO: 34 | |
| GB10-03 | Amino Acid Sequence | SEQ ID NO: 26 | SEQ ID NO: 27 | SEQ ID NO: 18 | SEQ ID NO: 30 |
| | Nucleotide Sequence | SEQ ID NO: 15 | SEQ ID NO: 40 | SEQ ID NO: 35 | |
| GB10-09 | Amino Acid Sequence | SEQ ID NO: 26 | SEQ ID NO: 27 | SEQ ID NO: 19 | SEQ ID NO: 31 |
| | Nucleotide Sequence | SEQ ID NO: 15 | SEQ ID NO: 40 | SEQ ID NO: 36 | |
| GB10-14 | Amino Acid Sequence | SEQ ID NO: 26 | SEQ ID NO: 27 | SEQ ID NO: 20 | SEQ ID NO: 32 |
| | Nucleotide Sequence | SEQ ID NO: 15 | SEQ ID NO: 40 | SEQ ID NO: 37 | |

TCCGATACAGGAAGGCCTTTTGTGGAAATGTACTCCGAGATCCCCGAGA

TCATCCACATGACCGAGGGCAGGGAACTGGTGATTCCTTGCAGGGTGACCTCCCC
CAATATCACCGTGACCCTGAAGAAGTTCCCCCTGGACACCCTGATCCCCGACGGA
AAAAGGATCATCTGGGACTCCAGGAAGGGCTTCATCATCTCCAACGCCACCTACA
AGGAGATCGGCCTGCTGACCTGTGAGGCCACAGTGAATGGCCATCTGTATAAGAC
CAACTACCTGACCCACAGGCAGACCAACACCATCATCGACGTGGTGCTGTCCCCC
TCCCATGGCATTGAGCTGTCTGTTGGCGAGAAACTGGTGCTGAACTGTACCGCTAG
GACCGAACTGAATGTGGGCATCGACTTTAACTGGGAGTACCCCTCCTCCAAGCAC
CAGCACAAAAAGCTGGTGAACAGGGACCTGAAGACCCAGTCCGGCTCCGAAATG
AAAAAGTTCCTGTCCACCCTGACCATCGACGGCGTGACAAGGTCCGATCAAGGCC
TGTATACCTGTGCTGCCTCCTCCGGCCTGATGACCAAAAAGAACTCCACCTTCGTG
AGGGTGCACGAGAAGGACAAGACCCACACCTGCCCCCCTTGTCCCGCTCCTGAAC
TGCTGGGAGGACCTTCTGTGTTTCTGTTTCCCCCTAAACCCAAAGATACCCTGATG
ATCTCCAGGACCCCCGAGGTGACATGTGTGGTGGTTGATGTGTCCCATGAAGACCC
CGAGGTGAAGTTCAACTGGTACGTGGACGGCGTGGAGGTGCACAATGCTAAGACC
AAGCCCAGGGAGGAGCAGTATAACTCCACCTACAGGGTGGTGTCCGTGCTGACCG
TGCTGCATCAAGACTGGCTGAACGGCAAGGAGTATAAGTGCAAGGTGTCCAACAA
GGCCCTGCCCGCCCCTATTGAAAAGACCATTTCCAAGGCCAAGGGCCAGCCCAGG
GAGCCTCAAGTTTATACACTGCCTCCCTCCAGGGACGAGCTGACAAAAAATCAGG
TGTCCCTGACCTGCCTGGTGAAGGGCTTTTACCCCTCCGACATCGCCGTGGAGTGG
GAATCCAATGGCCAGCCCGAAAACAACTACAAGACCACCCCCCCCGTGCTGGACT
CCGACGGGTCCTTTTTTCTGTACTCCAAGCTGACCGTGGACAAGTCCAGGTGGCA
GCAGGGAAATGTGTTCTCCTGCTCCGTGATGCACGAGGCCCTGCATAACCACTACA
CCCAGAAGTCCCTGTCCCTGTCCCCCGGA (SEQ ID NO: 15);

GGAGGAGGAGGATCTGGAGGAGGAGGGTCCGGAGGAGGAGGAAGCGG
AGGAGGAGGAAGC (SEQ ID NO: 40).

### Example 5: Affinity Measurement of Tetravalent Bispecific Inhibitor for VEGF and ANG-2

### 1. Affinity Measurement of Tetravalent Bispecific Antibody for Human VEGF-A165 and PLGF

The ProA sensor (Sartorius, 18-5010) was soaked in 1×KB solution for 10 min. The test samples (GB10-04, GB10-08, GB10-03, GB10-09 and GB10-14) obtained in Example 4 and the control drug Faricimab were diluted to 1 µg/mL with 1×KB solution. The prewetted sensors were transferred into 1×KB solution and equilibrated for 60 seconds, and then placed into the solutions containing the above proteins, allowing each protein to couple onto the ProA sensor. The capture endpoint was set to reach a Loading thickness of 1.0 nm, with a shaking speed of 1000 rpm/min. Human VEGFA165.his (ACRO, VE5-H5248) was diluted to 50 nmol/L using 1×KB and serially diluted in a two-fold gradient across eight concentrations in a black 96-well plate, with the lowest concentration set to 0 nmol/L. The sensors coupled with the proteins were re-equilibrated in 1×KB buffer for 120 seconds before being transferred to different concentrations of VEGFA165.his solutions for binding, with a shaking speed set to 1000 rpm/min. Then, the ProA sensors to which samples of different concentrations bound were transferred into 1×KB for dissociation, with a shaking speed set to 1000 rpm/min. After dissociation, the sensors were sequentially placed in a 10 mmol/L glycine solution and 1×KB for 5 seconds, with a shaking speed set to 1000 rpm/min. This process was repeated three times to ensure complete elution of complexes from the ProA sensor before testing the next sample.

The affinity measurement results are shown in Table 4. The tetravalent bispecific antibody proteins GB10-04, GB10-08, GB10-03, GB10-09, and GB10-14 exhibited higher binding affinity to VEGF compared to Faricimab.

**Table 4: VEGF Affinity of Tetravalent Bispecific Antibody Proteins**

| Sample Name | Antigen | K_{D} (M) | Kon (1/Ms) | Kdis (1/s) |
|---|---|---|---|---|
| GB10-04 | VEGF | 9.54E-10 | 4.07E+05 | 3.88E-04 |
| GB10-08 | | 4.92E-10 | 2.27E+05 | 1.11E-04 |
| GB10-03 | | 8.84E-10 | 2.54E+04 | 2.24E-05 |
| GB10-09 | | 9.54E-10 | 4.07E+05 | 3.88E-04 |
| GB10-14 | | 1.29E-10 | 1.47E+05 | 1.90E-05 |
| Faricimab | | 1.53E-09 | 5.42E+05 | 8.27E-04 |

### 2. Affinity Measurement of Tetravalent Bispecific Antibody for Human ANG-2

The NTA sensor (Sartorius, 18-5101) was soaked in 1×KB solution for 10 min. Human ANG-2.his protein (Sino, 10691-H08H) was diluted to 1 µg/mL with 1×KB solution. The prewetted sensors were transferred into 1×KB solution and equilibrated for 60 seconds, and then placed into the human ANG-2.his solution, allowing human ANG-2.his to couple onto the NTA sensor. The capture endpoint was set to reach a Loading thickness of 1.0 nm, with a shaking speed of 1000 rpm/min. The test samples (GB10-04, GB10-08, GB10-03, GB10-09 and GB10-14) prepared in Example 4 and the control drug Faricimab was diluted to 30 nmol/L using 1×KB and serially diluted in a two-fold gradient across eight concentrations in a black 96-well plate, with the lowest concentration set to 0 nmol/L. The sensors coupled with human ANG-2.his were re-equilibrated in 1×KB buffer for 120 seconds before being transferred to different concentrations of test sample solutions for binding, with a shaking speed set to 1000 rpm/min. Then, the NTA sensors to which samples of different concentrations bound were transferred into 1×KB for dissociation, with a shaking speed set to 1000 rpm/min. After dissociation, the sensors were sequentially placed in a 10 mmol/L glycine solution and 1×KB for 5 seconds, with a shaking speed set to 1000 rpm/min. This process was repeated three times to ensure complete elution of complexes from the NTA sensor, which was then transferred into a 10 mmol/L NiCl₂ solution to activate for 60 seconds before testing the next sample. The affinity measurement results are shown in Table 5. GB10-04, GB10-08, GB10-03, GB10-09, and GB10-14 exhibited higher binding affinity to ANG-2 compared to Faricimab.

**Table 5: ANG-2Affinity of Tetravalent Bispecific Antibody Proteins**

| Sample name | Antigen | K_{D} (M) | Kon (1/Ms) | Kdis (1/s) |
|---|---|---|---|---|
| GB10-04 | ANG-2 | <1.0E-12 | 1.06E+06 | <1.0E-07 |
| GB10-08 | | <1.0E-12 | 3.25E+05 | <1.0E-07 |
| GB10-03 | | <1.0E-12 | 2.06E+05 | <1.0E-07 |
| GB10-09 | | <1.0E-12 | 1.44E+05 | <1.0E-07 |
| GB10-14 | | <1.0E-12 | 3.54E+04 | <1.0E-07 |
| Faricimab | | 3.44E-09 | 1.08E+06 | 3.71E-03 |

### Example 6: Measurement of VEGF Blocking Activity of Tetravalent Bispecific Inhibitor

### 1. Measurement of VEGF Reporter Gene

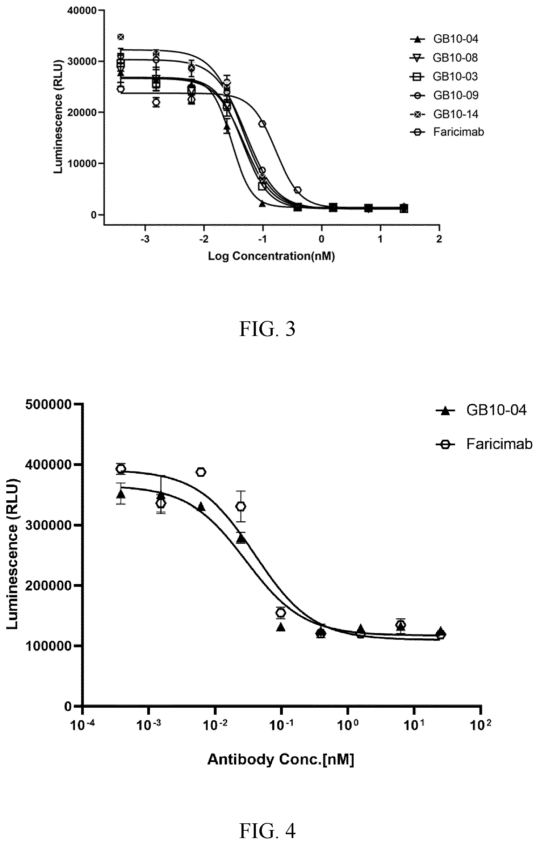

A H-VEGF Reporter 293 cell (GM-C09057) suspension was added to a 96-well microplate (BeyoGold, FCP968) at 2.5×10⁴ cells/50 µL/well and cultured overnight in a carbon dioxide incubator. The microplate was taken out. Each well received 50 µL of a mixture containing VEGF165 (having a final concentration of 5 ng/mL, Novoprotein, C083) as well as test samples (GB10-04, GB10-08, GB10-03, GB10-09 or GB10-14 prepared in Example 4) diluted in different gradients (with a highest concentration of 25 nM, followed by a 3-fold serial dilution across eight gradients) or the control drug (Faricimab). The cells were further cultured in an incubator at 37°C with 5% CO₂. After 6 h, 100 µL of luciferase reporter detection reagent (Vazyme, DD1203) was added to each well. The plate was incubated for 15 minutes before measuring the relative luminescence units (RLU). GraphPad Prism 8.0 software was used to analyze data for fitting of the inhibition activity to obtain an IC₅₀ value of VEGF blocking activity. The results are shown in Table 6 and FIG. 3. The results show that GB10-04, GB10-08, GB10-03, GB10-09, GB10-14 and Faricimab exhibited obvious blocking activity against the expression of VEGF-mediated reporter gene. Notably, the VEGF blocking activity of the tetravalent bispecific inhibitors of the present disclosure was superior to that of Faricimab.

**Table 6: IC₅₀ Values for VEGF Reporter Gene Inhibition**

| Sample | IC₅₀ (nM) | Relative Activity |
|---|---|---|
| GB10-04 | 0.030 | 557% |
| GB10-08 | 0.048 | 347% |
| GB10-03 | 0.045 | 366% |
| GB10-09 | 0.051 | 322% |
| GB10-14 | 0.045 | 371% |
| Faricimab | 0.165 | 100% |

### 2. HUVEC proliferation activity detection

AHUVEC cell (Promocell #C12205) suspension was added to a 96-well microplate (Corning, 3917) at 3000 cells/50 µL/well and cultured overnight in a carbon dioxide incubator. The microplate was taken out. Each well received 50 µL of a mixture containing VEGF165 (having a final concentration of 5 ng/mL, Novoprotein, C083) as well as test samples (GB10-04, GB10-08, GB10-03, GB10-09 or GB10-14 prepared in Example 4) diluted in different gradients (with a highest concentration of 25 nM, followed by a 3-fold serial dilution across eight gradients) or the control drug (Faricimab). The cells were further cultured in an incubator at 37°C with 5% CO₂. After 3 days, cell proliferation was measured. 50 µL of Cell Titer-Glo^{®} Luminescent Cell Viability Assay reagent (Promega, G7570) was added to each well. The plate was gently shaken and incubated for 15 minutes before measuring RLU. GraphPad Prism 8.0 software was used to analyze data for fitting of the inhibition activity to obtain an IC₅₀ value of HUVEC proliferation inhibition activity. The results are shown in Table 6 and FIG. 4. The results show that GB10-04, GB10-08, GB10-03, GB10-09 and GB10-14 exhibited significant blocking activity against VEGF-mediated HUVEC proliferation. Among them, GB10-04 exhibited superior VEGF blocking activity compared to Faricimab,

**Table 7: IC₅₀ Values for HUVEC Proliferation Inhibition**

| Sample | IC₅₀ (nM) |
|---|---|
| GB10-04 | 0.029 |
| Faricimab | 0.040 |

### Example 7: Measurement of ANG-2 Blocking Activity of Tetravalent Bispecific Inhibitor

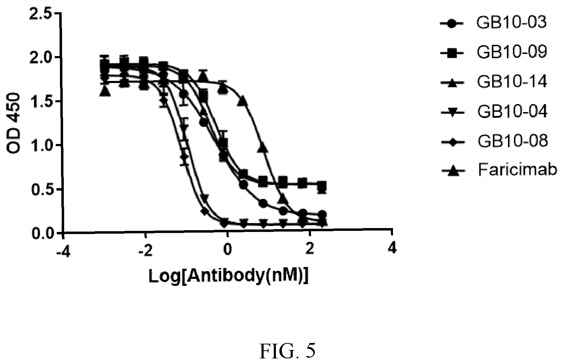

TIE2 protein (Acro, TI2-H5255) was diluted to prepare a 1 µg/mL coating solution, which was added to an ELISA plate at 100 µL/well and coated at 2-8°C for more than 12 h. The residual coating solution was discarded, and 200 µL of 2% BSA-PBS was added per well for blocking at 37°C for 2 hours, followed by three washes with 300 µL of PBST per well. ANG-2 protein (Sino Biological, 10691-H08H) was prepared at 100 ng/mL in sample dilution solutions. The test samples (GB10-04, GB10-08, GB10-03, GB10-09, and GB10-14 prepared in Example 4) and the control drug (Faricimab) was diluted to 20 nM, then serially diluted in a three-fold gradient across eight concentrations, added to the ELISA plate at 100 µL/well, and incubated at 37°C for 1 hour, followed by three washes with 300 µL of PBST per well. Goat anti-Human His-HRP antibody (Abcam, ab1187) diluted in 1:10,000 was added at 100 µL/well, and incubated at 37°C for 1 hour, followed by three washes with 300 µL of PBST per well. 100 µL of TMB substrate solution was added per well and incubated at room temperature for 5 minutes. The reaction was stopped with 50 µL of 2M H₂SO₄ per well. The ELISA plate was then placed on a microplate reader, and the absorbance at 450 nm (OD₄₅₀) was read. GraphPad Prism 7.0 software was used to analyze data for fitting of the inhibitory activity to obtain an IC₅₀ value of ANG-2 inhibitory activity. The results are shown in Table 8 and FIG. 5. The results showed that GB10-04, GB10-08, GB10-03, GB10-09 and GB10-14 exhibited significant blocking activity against the binding of ANG-2 to its receptor TIE2. Moreover, all five tetravalent bispecific inhibitors demonstrated superior ANG-2 blocking activity compared to Faricimab.

**Table 8. IC₅₀ Values for ANG-2 Blocking**

| Sample | IC₅₀ (nM) | Relative Activity |
|---|---|---|
| GB10-04 | 0.112 | 7069% |
| GB10-08 | 0.081 | 9775% |
| GB10-03 | 0.518 | 1528% |
| GB10-09 | 0.533 | 1485% |
| GB10-14 | 0.396 | 1999% |
| Faricimab | 7.918 | 100% |

In the description of the present specification, references to the terms such as "an embodiment", "some embodiments", "an example", "a specific example" and "some examples" indicate that the specific features, structures, materials or characteristics described in conjunction with the embodiment or example are included in at least one embodiment or example of the present disclosure. In this specification, the schematic representation of the above terms does not necessarily refer to the same embodiment or example. Moreover, the specific features, structures, materials or characteristics described can be combined in any one or more embodiments or examples in a suitable manner. In addition, those skilled in the art can combine and integrate different embodiments or examples described in this specification and the features of different embodiments or examples without contradiction.
Although the embodiments of the present disclosure have been shown and described above, it can be understood that the above embodiments are exemplary and cannot be understood as limitations of the present invention. Those skilled in the art may make adjustments, modifications, substitutions, or variations to the above embodiments within the scope of the present disclosure.

## Claims

1. An antibody or antigen-binding fragment, comprising at least one heavy chain variable region CDR sequence selected from SED ID NO: 1 to SED ID NO: 14 or an amino acid sequence having at least 80% identity to SED ID NO: 1 to SED ID NO: 14.

2. The antibody or antigen-binding fragment according to claim 1, comprising:
a heavy chain variable region CDR1 sequence, a heavy chain variable region CDR2 sequence, and a heavy chain variable region CDR3 sequence specified in amino acid sequences SEQ ID NO: 1, SED ID NO: 2, and SED ID NO: 3, respectively, or with amino acid sequences having at least 80% identity to SEQ ID NO: 1, SED ID NO: 2, and SED ID NO: 3, respectively;
a heavy chain variable region CDR1 sequence, a heavy chain variable region CDR2 sequence, and a heavy chain variable region CDR3 sequence specified in amino acid sequences SEQ ID NO: 4, SED ID NO: 5, and SED ID NO: 6, respectively, or with amino acid sequences having at least 80% identity to SEQ ID NO: 4, SEQ ID NO: 5, and SEQ ID NO: 6, respectively;
a heavy chain variable region CDR1 sequence, a heavy chain variable region CDR2 sequence, and a heavy chain variable region CDR3 sequence specified in amino acid sequences SEQ ID NO: 7, SED ID NO: 8, and SED ID NO: 9, respectively, or with amino acid sequences having at least 80% identity to SEQ ID NO: 7, SEQ ID NO: 8, and SEQ ID NO: 9, respectively;
a heavy chain variable region CDR1 sequence, a heavy chain variable region CDR2 sequence, and a heavy chain variable region CDR3 sequence specified in amino acid sequences SEQ ID NO: 10, SED ID NO: 11, and SED ID NO: 12, respectively, or with amino acid sequences having at least 80% identity to SEQ ID NO: 10, SED ID NO: 11, and SED ID NO: 12, respectively; or
a heavy chain variable region CDR1 sequence, a heavy chain variable region CDR2 sequence, and a heavy chain variable region CDR3 sequence specified in amino acid sequences SEQ ID NO: 10, SED ID NO: 13, and SED ID NO: 14, respectively, or with amino acid sequences having at least 80% identity to SEQ ID NO: 10, SED ID NO: 13, and SED ID NO: 14, respectively.

3. The antibody or antigen-binding fragment according to claim 1, wherein the antibody comprises a heavy chain framework region sequence, wherein at least a part of the heavy chain framework region sequence is derived from at least one of murine antibody, human antibody, primate antibody, alpaca antibody, or a variant thereof.

4. The antibody or antigen-binding fragment according to claim 1, wherein the antibody has a heavy chain variable region with an amino acid sequence specified in any one of SEQ ID NO: 16 to SEQ ID NO: 20.

5. The antibody or antigen-binding fragment according to claim 1, wherein the antibody comprises a heavy chain constant region, wherein at least a part of the heavy chain constant region is derived from at least one of murine antibody, human antibody, primate antibody, or a variant thereof.

6. The antibody or antigen-binding fragment according to claim 5, wherein the heavy chain constant region of the antibody is derived from a human IgG antibody or a variant thereof.

7. The antibody or antigen-binding fragment according to claim 6, wherein the heavy chain constant region of the antibody is derived from human IgG1.

8. The antibody or antigen-binding fragment according to claim 1, wherein the antibody is a monoclonal antibody or a polyclonal antibody.

9. The antibody or antigen-binding fragment according to claim 8, wherein the monoclonal antibody comprises at least one of Fab antibody, Fv antibody, single-chain antibody, single-domain antibody, and minimal recognition unit.

10. The antibody or antigen-binding fragment according to claim 1, wherein the antibody has a heavy chain with an amino acid sequence specified in any one of SEQ ID NO: 21 to SEQ ID NO: 25.

11. A recombinant protein, comprising the antibody or antigen-binding fragment according to any one of claims 1 to 10.

12. The recombinant protein according to claim 11, further comprising at least one selected from a bioactive protein or fragment thereof, and a bioactive polypeptide or fragment thereof.

13. The recombinant protein according to claim 12, wherein the bioactive protein or fragment thereof comprises at least one selected from a protein tag, a protein toxin or fragment thereof, a tumor necrosis factor or fragment thereof, an interferon or fragment thereof, a biological response regulator or fragment thereof, and an Fc fragment.

14. A multispecific antibody, comprising:
a first binding region, comprising the antibody or antigen-binding fragment according to any one of claims 1 to 10; and
a second binding region, having an activity of binding to a first molecule.

15. The multispecific antibody according to claim 14, comprising at least one selected from a bispecific antibody, a trispecific antibody, and a tetraspecific antibody, preferably a bispecific antibody.

16. The multispecific antibody according to claim 15, wherein the first molecule comprises at least one selected from immune cell surface antigen, tumor antigen, virus, bacteria, endotoxin, cytokine, and cytokine receptor.

17. The multispecific antibody according to claim 16, wherein the first molecule comprises at least one selected from PD-L1, PD-1, IL-10, IL-10R, BCMA, VEGF, TGF-β, CTLA-4, LAG-3, TIGIT, CEA, CD38, SLAMF7, B7-H3, Her2, EpCAM, CD19, CD20, CD30, CD33, CD47, CD52, CD133, EGFR, GD2, GD3, GM2, RANKL, CD3, and CD16a.

18. The multispecific antibody according to claim 14, wherein the second binding region is a binding protein or fragment thereof to the first molecule.

19. The multispecific antibody according to claim 14, wherein the first molecule is VEGF.

20. The multispecific antibody according to claim 19, wherein the second binding region comprises at least one selected from a VEGF receptor fragment and an anti-VEGF antibody.

21. The multispecific antibody according to claim 20, wherein the VEGF receptor fragment comprises at least one selected from a VEGFR1 fragment, a VEGFR2 fragment, and a VEGFR3 fragment.

22. The multispecific antibody according to claim 21, wherein the second binding region comprises an immunoglobulin-like domain 2 of the VEGFR1 fragment and/or an immunoglobulin-like domain 3 of the VEGFR2 fragment.

23. The multispecific antibody according to claim 22, wherein the C-terminus of immunoglobulin-like domain 2 of the VEGFR1 fragment is linked to the N-terminus of immunoglobulin-like domain 3 of the VEGFR2 fragment.

24. The multispecific antibody according to claim 23, wherein the bispecific antibody is a symmetric bispecific antibody or an asymmetric bispecific antibody.

25. The multispecific antibody according to claim 23, wherein the bispecific antibody is a symmetric bispecific antibody.

26. The multispecific antibody according to claim 25, wherein the second binding region further comprises an Fc fragment.

27. The multispecific antibody according to claim 26, wherein:
the C-terminus of immunoglobulin-like domain 2 of the VEGFR1 fragment is linked to the N-terminus of immunoglobulin-like domain 3 of the VEGFR2 fragment, and the C-terminus of immunoglobulin-like domain 3 of the VEGFR2 fragment is linked to the N-terminus of the Fc fragment; or
the C-terminus of immunoglobulin-like domain 2 of the VEGFR1 fragment is linked to the N-terminus of immunoglobulin-like domain 3 of the VEGFR2 fragment, and the N-terminus of immunoglobulin-like domain 2 of the VEGFR1 fragment is linked to the C-terminus of the Fc fragment.

28. The multispecific antibody according to claim 14, wherein the second binding region has an amino acid sequence specified in SEQ ID NO: 26 or an amino acid sequence having at least 90% identity to SEQ ID NO: 26.

29. The multispecific antibody according to claim 27, further comprising a linking peptide.

30. The multispecific antibody according to claim 29, wherein:
the C-terminus of immunoglobulin-like domain 3 of the VEGFR2 fragment is linked to the N-terminus of the Fc fragment, the C-terminus of the Fc fragment is linked to the N-terminus of the linking peptide, and the C-terminus of the linking peptide is linked to the N-terminus of the antibody or antigen-binding fragment; or
the C-terminus of the antibody or antigen-binding fragment is linked to the N-terminus of the linking peptide, the C-terminus of the linking peptide is linked to the N-terminus of the Fc fragment, and the C-terminus of the Fc fragment is linked to the N-terminus of immunoglobulin-like domain 2 of the VEGFR1 fragment.

31. The multispecific antibody according to claim 29, wherein the amino acid sequence of the linking peptide is (GGGGS)ₙ, n being an integer greater than or equal to 1, preferably 1, 2, 3, 4, 5, 6, 7, 8, 9, or 10.

32. The multispecific antibody according to claim 29, wherein the linking peptide has an amino acid sequence specified in SEQ ID NO: 27.

33. The multispecific antibody according to claim 14, wherein the antibody has an amino acid sequence specified in any one of SEQ ID NO: 28 to SEQ ID NO: 32.

34. A nucleic acid molecule, encoding the antibody or antigen-binding fragment according to any one of claims 1 to 10, the recombinant protein according to any one of claims 11 to 13, or the multispecific antibody according to any one of claims 14 to 33.

35. The nucleic acid molecule according to claim 34, wherein the nucleic acid molecule is DNA.

36. An expression vector, carrying the nucleic acid molecule according to claim 34 or 35.

37. The expression vector according to claim 36, wherein the expression vector is a eukaryotic expression vector, preferably a plasmid expression vector.

38. A recombinant cell,
the recombinant cell carrying the nucleic acid molecule according to claim 34 or 35 or the expression vector according to claim 36 or 37; or
the recombinant cell expressing the antibody or antigen-binding fragment according to any one of claims 1 to 10, the recombinant protein according to any one of claims 11 to 13, or the multispecific antibody according to any one of claims 14 to 33.

39. The recombinant cell according to claim 38, wherein the recombinant cell is obtained by introducing the expression vector according to claim 36 or 37 into a host cell.

40. The recombinant cell according to claim 38, wherein the recombinant cell is a eukaryotic cell.

41. The recombinant cell according to claim 38, wherein the recombinant cell is a mammalian cell.

42. A pharmaceutical composition, comprising the antibody or antigen-binding fragment according to any one of claims 1 to 10, the recombinant protein according to any one of claims 11 to 13, the multispecific antibody according to any one of claims 14 to 33, the nucleic acid molecule according to claim 34 or 35, the expression vector according to claim 36 or 37, or the recombinant cell according to any one of claims 38 to 41.

43. The pharmaceutical composition according to claim 42, further comprising a pharmaceutically acceptable excipient.

44. A conjugate, comprising:
the antibody or antigen-binding fragment according to any one of claims 1 to 10, the recombinant protein according to any one of claims 11 to 13, or the multispecific antibody according to any one of claims 14 to 33; and
a conjugation moiety linked to the antibody or antigen-binding fragment, the recombinant protein, or the multispecific antibody.

45. The conjugate according to claim 44, wherein the conjugation moiety comprises at least one selected from a carrier, drug, toxin, cytokine, protein tag, modifier, and chemotherapeutic agent.

46. A kit, comprising the antibody or antigen-binding fragment according to any one of claims 1 to 10, the recombinant protein according to any one of claims 11 to 13, the multispecific antibody according to any one of claims 14 to 33, the nucleic acid molecule according to claim 34 or 35, the expression vector according to claim 36 or 37, the recombinant cell according to any one of claims 38 to 41, or the conjugate according to claim 44 or 45.

47. Use of the antibody or antigen-binding fragment according to any one of claims 1 to 10, the recombinant protein according to any one of claims 11 to 13, the multispecific antibody according to any one of claims 14 to 33, the nucleic acid molecule according to claim 34 or 35, the expression vector according to claim 36 or 37, the recombinant cell according to any one of claims 38 to 41, or the conjugate according to claim 44 or 45, in the preparation of a kit for detecting ANG-2.

48. The use according to claim 47, wherein the kit is further used for detecting VEGF.

49. Use of the antibody or antigen-binding fragment according to any one of claims 1 to 10, the recombinant protein according to any one of claims 11 to 13, the multispecific antibody according to any one of claims 14 to 33, the nucleic acid molecule according to claim 34 or 35, the expression vector according to claim 36 or 37, the recombinant cell according to any one of claims 38 to 41, the pharmaceutical composition according to claim 42 or 43, or the conjugate according to claim 44 or 45, in the manufacture of a medicament for treating or preventing an angiogenesis-related disease.

50. Use of the antibody or antigen-binding fragment according to any one of claims 1 to 10, the recombinant protein according to any one of claims 11 to 13, the multispecific antibody according to any one of claims 14 to 33, the nucleic acid molecule according to claim 34 or 35, the expression vector according to claim 36 or 37, the recombinant cell according to any one of claims 38 to 41, the pharmaceutical composition according to claim 42 or 43, or the conjugate according to claim 44 or 45, in the treatment or prevention of an angiogenesis-related disease.

51. Use of the antibody or antigen-binding fragment according to any one of claims 1 to 10, the recombinant protein according to any one of claims 11 to 13, the multispecific antibody according to any one of claims 14 to 33, the nucleic acid molecule according to claim 34 or 35, the expression vector according to claim 36 or 37, the recombinant cell according to any one of claims 38 to 41, the conjugate according to claim 44 or 45, or the kit according to claim 46, in the diagnosis, prognosis assessment, or staging of an angiogenesis-related disease.

52. The use according to any one of claims 49 to 51, wherein the angiogenesis-related disease comprises at least one selected from cancer, ophthalmic disease, and inflammatory disease.

53. A method for treating or preventing an angiogenesis-related disease, comprising:
administering to a subject a pharmaceutically acceptable amount of the antibody or antigen-binding fragment according to any one of claims 1 to 10, the recombinant protein according to any one of claims 11 to 13, the multispecific antibody according to any one of claims 14 to 33, the pharmaceutical composition according to claim 42 or 43, or the conjugate according to claim 44 or 45.

54. The method according to claim 53, wherein a route of said administering is subcutaneous injection or intravenous injection.

55. A method for diagnosing an angiogenesis-related disease, comprising:
detecting ANG-2 in a test sample using the antibody or antigen-binding fragment according to any one of claims 1 to 10, the recombinant protein according to any one of claims 11 to 13, the multispecific antibody according to any one of claims 14 to 33, the nucleic acid molecule according to claim 34 or 35, the expression vector according to claim 36 or 37, the recombinant cell according to any one of claims 38 to 41, the conjugate according to claim 44 or 45, or the kit according to claim 46; and
determining a content of ANG-2 in the test sample based on a detection result.

56. The method according to claim 55, further comprising:
detecting VEGF in the test sample; and
determining a content of VEGF in the test sample based on a detection result.

57. A method for prognosis assessment of an angiogenesis-related disease, comprising:
detecting ANG-2 in a test sample using the antibody or antigen-binding fragment according to any one of claims 1 to 10, the recombinant protein according to any one of claims 11 to 13, the multispecific antibody according to any one of claims 14 to 33, the nucleic acid molecule according to claim 34 or 35, the expression vector according to claim 36 or 37, the recombinant cell according to any one of claims 38 to 41, the conjugate according to claim 44 or 45, or the kit according to claim 46; and
determining a content of ANG-2 in the test sample based on a detection result.

58. The method according to claim 57, further comprising:
detecting VEGF in the test sample; and
determining a content of VEGF in the test sample based on a detection result.

59. A method for staging an angiogenesis-related disease, comprising:
detecting ANG-2 in a test sample using the antibody or antigen-binding fragment according to any one of claims 1 to 10, the recombinant protein according to any one of claims 11 to 13, the multispecific antibody according to any one of claims 14 to 33, the nucleic acid molecule according to claim 34 or 35, the expression vector according to claim 36 or 37, the recombinant cell according to any one of claims 38 to 41, the conjugate according to claim 44 or 45, or the kit according to claim 46; and
determining a content of ANG-2 in the test sample based on a detection result.

60. The method according to claim 59, further comprising:
detecting VEGF in the test sample; and
determining a content of VEGF in the test sample based on a detection result.

61. The method according to claim 60, wherein:
the content of ANG-2 and/or the content of VEGF in the test sample not lower than the minimum level for a stage IV tumor indicates that the test sample is derived from a patient with a stage IV tumor;
the content of ANG-2 and/or the content of VEGF in the test sample between a level for a stage **III** tumor and a level for a stage IV tumor indicates that the test sample is derived from a patient with a stage III tumor;
the content of ANG-2 and/or the content of VEGF in the test sample falling between a level for a stage II tumor and a level for a stage III tumor indicates that the test sample is derived from a patient with a stage II tumor; and
the content of ANG-2 and/or the content of VEGF in the test sample falling between a level for a stage I tumor and a level for a stage II tumor indicates that the test sample is derived from a patient with a stage I tumor.

62. The method according to any one of claims 53 to 61, wherein the angiogenesis-related disease comprises at least one selected from cancer, ophthalmic disease, and inflammatory disease.

63. The antibody or antigen-binding fragment according to any one of claims 1 to 10, the recombinant protein according to any one of claims 11 to 13, the multispecific antibody according to any one of claims 14 to 33, the nucleic acid molecule according to claim 34 or 35, the expression vector according to claim 36 or 37, the recombinant cell according to any one of claims 38 to 41, the pharmaceutical composition according to claim 42 or 43, or the conjugate according to claim 44 or 45, for use in the treatment or prevention of an angiogenesis-related disease.

64. The antibody or antigen-binding fragment according to any one of claims 1 to 10, the recombinant protein according to any one of claims 11 to 13, the multispecific antibody according to any one of claims 14 to 33, the nucleic acid molecule according to claim 34 or 35, the expression vector according to claim 36 or 37, the recombinant cell according to any one of claims 38 to 41, the pharmaceutical composition according to claim 42 or 43, or the conjugate according to claim 44 or 45, for use in the diagnosis, prognosis assessment, or staging of an angiogenesis-related disease.
